# EUROPÄISCHE PATENTSCHRIFT

(11) **EP 4 178 472 B1**
(45) Veröffentlichungstag und Bekanntmachung des Hinweises auf die Patenterteilung: **20.09.2023**
(21) Anmeldenummer: 22705765.0
(22) Anmeldetag: 09.02.2022
(51) Int. Cl.: A61B 17/29, A61B 17/00

(54) **CHIRURGISCHES INSTRUMENT, WERKZEUGEINRICHTUNG FÜR EIN SOLCHES CHIRURGISCHES INSTRUMENT UND VERFAHREN ZUR HERSTELLUNG EINER SOLCHEN WERKZEUGEINRICHTUNG**
SURGICAL INSTRUMENT, TOOL DEVICE FOR SUCH A SURGICAL INSTRUMENT, AND METHOD FOR PRODUCING SUCH A TOOL DEVICE
INSTRUMENT CHIRURGICAL, DISPOSITIF D'OUTIL POUR UN TEL INSTRUMENT CHIRURGICAL ET PROCÉDÉ DE FABRICATION D'UN TEL DISPOSITIF D'OUTIL

(30) Priorität: 11.02.2021 DE 102021201311
(43) Veröffentlichungstag der Anmeldung: 17.05.2023
(73) Patentinhaber: Aesculap AG, 78532 Tuttlingen (DE)
(72) Erfinder: HERNER, Eugen, 78054 Villingen-Schwenningen (DE); KIZENBERGER, Hannes, 78194 Immendingen (DE); HAFNER, Nikolaus, 78532 Tuttlingen (DE); WALBERG, Erik, 86150 Augsburg (DE)
(74) Vertreter: Patentanwälte Ruff, Wilhelm, Beier, Dauster & Partner mbB
(86) Internationale Anmeldenummer: PCT/EP2022/053124
(87) Internationale Veröffentlichungsnummer: WO 2022/171668

(56) Entgegenhaltungen:
- EP-B1- 2 688 501
- DE-A1- 10 136 963
- US-A1- 2020 179 038

## Beschreibung

Die Erfindung betrifft eine Werkzeugeinrichtung für ein chirurgisches Instrument mit den oberbegrifflichen Merkmalen des Anspruchs 1.

Eine derartige Werkzeugeinrichtung ist aus der EP 2 688 501 B1 bekannt und weist zwei Maulteile auf, wobei eines der beiden Maulteile beweglich gelagert ist und durch einen Stift-Nut-Mechanismus geöffnet und geschlossen werden kann.

Weiter ist aus der US 2020/0179038 A1 ein chirurgisches Instrument bekannt und in Form eines endoskopischen Zangeninstruments zum Versiegeln und/oder Durchtrennen von Körpergewebe vorgesehen. Das bekannte chirurgische Instrument weist einen längserstreckten Instrumentenschaft, eine Griffeinrichtung mit einem Bedienelement und eine Werkzeugeinrichtung mit einem öffenbaren und schließbaren Werkzeugmaul auf. Die Werkzeugeinrichtung ist an einem distalen Ende des Instrumentenschafts angeordnet. Die Griffeinrichtung ist an einem proximalen Ende des Instrumentenschafts angeordnet. Das Bedienelement ist über ein in dem Instrumentenschaft längserstrecktes und translatorisch verlagerbares Zug-Druck-Element zum Öffnen und/oder Schließen des Werkzeugmauls mit der Werkzeugeinrichtung wirkverbunden. Hierzu greift das Zug-Druck-Element an einem Steuerstift einer Steueranordnung der Werkzeugeinrichtung an. Die Steueranordnung des bekannten chirurgischen Instruments weist eine an dem ersten Maulteil ausgebildete erste Steuernut und eine an dem zweiten Maulteil ausgebildete zweite Steuernut auf. Der Steuerstift durchgreift beide Steuernuten und ist mittels einer Längsbewegung des Zug-Druck-Elements zwischen einer proximalen und einer distalen Stiftendlage entlang der Steuernuten gleitbeweglich verlagerbar. Hierdurch wird die Längsbewegung des Zug-Druck-Elements in eine um die Schwenkachse gerichtete Schwenkbewegung der beiden Maulteile zum Öffnen und/oder Schließen des Werkzeugmauls übersetzt.

Weiter ist aus der DE 101 36 963 A1 ein chirurgisches Instrument mit einer Werkzeugeinrichtung mit zwei Maulteilen bekannt, bei welcher nur eines der beiden Maulteile beweglich gelagert ist.

Aufgabe der Erfindung ist es, eine Werkzeugeinrichtung der eingangs genannten Art bereitzustellen, die Vorteile gegenüber dem Stand der Technik bietet. Insbesondere sollen ein vereinfachter Aufbau der Werkzeugeinrichtung und damit einhergehend eine einfache Herstellung ermöglicht werden.

Diese Aufgabe wird durch das Bereitstellen einer Werkzeugeinrichtung mit den Merkmalen des Anspruchs 1 gelöst. Durch die erfindungsgemäße Lösung kann auf eine an dem zweiten Maulteil angeordnete und/oder ausgebildete Steuernut verzichtet werden. Solche Steuernuten werden üblicherweise als Langloch mittels Fräsens hergestellt. Dies ist vergleichsweise aufwändig. Anstelle einer üblichen Steuernut weist das zweite Maulteil den wenigstens einen Steuervorsprung auf, der von einer Innenwandung der Aufnahmeaussparung in Axialrichtung des Steuerstifts nach innen abragt und eine Steuerfläche der Steueranordnung ausbildet. Hierdurch können ein vergleichsweise einfacher Aufbau und eine einfache Herstellung der Werkzeugeinrichtung und damit einhergehend des chirurgischen Instruments erreicht werden. Das chirurgische Instrument ist vorzugsweise zum Greifen, Halten, Klemmen, Versiegeln und/oder Durchtrennen von Körpergewebe bei einem minimalinvasiven, insbesondere laparoskopischen, Eingriff vorgesehen. Dementsprechend ist die Werkzeugeinrichtung vorzugsweise zangen-, klemmen- und/oder scherenartig gestaltet, wobei das erste Maulteil und das zweite Maulteil ein Zangen-, Klemmen- und/oder Scherenmaul ausbilden. Die Maulteile können auch als Instrumenten- oder Gewebebranchen bezeichnet werden. Um bestimmungsgemäß Druck auf zwischen dem ersten Maulteil und dem zweiten Maulteil befindliches Körpergewebe ausüben zu können, sind das erste und zweite Maulteil um die Schwenkachse relativ zueinander schwenkbeweglich. Hierfür können beide Maulteile in Bezug auf die Schwenkachse schwenkbeweglich sein. Alternativ kann das erste Maulteil schwenkbeweglich und das zweite Maulteil feststehend sein oder umgekehrt. Die Steueranordnung dient einer Übersetzung der entlang der Längsbewegungsachse gerichteten translatorischen Bewegung des Zug-Druck-Elements in die um die Schwenkachse gerichtete Schwenkbewegung des ersten und/oder zweiten Maulteils. Die Längsbewegungsachse ist vorzugsweise parallel zur Schaftlängsachse orientiert. Die Schwenkachse ist vorzugsweise orthogonal zur Längsbewegungsachse und/oder der Schaftlängsachse orientiert. Die Steuernut des ersten Maulteils ist vorzugsweise relativ zu der Längsbewegungsachse geneigt längserstreckt. Sofern das erste Maulteil um die Schwenkachse schwenkbeweglich ist, ist ein entsprechender Neigungswinkel der Steuernut in Abhängigkeit einer Schwenkstellung des ersten Maulteils relativ zu der Längsbewegungsachse veränderlich. Die durch den wenigstens einen Steuervorsprung des zweiten Maulteils ausgebildete Steuerfläche kann parallel und/oder wenigstens abschnittsweise geneigt zu der Längsbewegungsachse längserstreckt sein. Vorzugsweise weist das zweite Maulteil in Axialrichtung des Steuerstifts beidseits der Aufnahmeaussparung angeordnete Steuervorsprünge auf. Das zweite Maulteil kann einstückig oder mehrstückig gestaltet sein. In letztgenanntem Fall weist das zweite Maulteil vorzugsweise einen Wirkabschnitt, der zum Einwirken auf das Körpergewebe vorgesehen ist, und einen die Aufnahmeaussparung mitsamt des wenigstens einen Steuervorsprungs tragenden Gehäuseabschnitt auf, wobei der Wirkabschnitt und der Gehäuseabschnitt jeweils separat gefertigt und hiernach, vorzugsweise formschlüssig, miteinander verbunden sind.

In Ausgestaltung der Erfindung weist das zweite Maulteil die Aufnahmeaussparung in Axialrichtung des Steuerstifts begrenzende Sicherungsflächen auf, zwischen welchen der Steuerstift bei einer Verlagerung zwischen der proximalen und der distalen Stiftendlage gegen eine axiale Verschiebung formschlüssig gesichert ist. Hierdurch kann auf eine gesonderte kraft- und/oder stoffschlüssige Verbindung des Steuerstifts mit dem Zug-Druck-Element zur axialen Sicherung verzichtet werden. Dies ermöglicht einen nochmals vereinfachten Aufbau und eine weiter vereinfachte Herstellung. Die Sicherungsflächen sind an der Innenwandung der Aufnahmeaussparung ausgebildet. Bei dieser Ausgestaltung der Erfindung ist die Aufnahmeaussparung in Axialrichtung des Steuerstifts beidseits geschlossen. Bei der Verlagerung zwischen der proximalen und der distalen Stiftendlange bewegt sich der Steuerstift innerhalb der Aufnahmeaussparung, entlang des Steuervorsprungs und in Bezug auf seine Axialrichtung zwischen den gegenüberliegenden Sicherungsflächen.

In weiterer Ausgestaltung der Erfindung weist das zweite Maulteil eine Schwenklagerfläche auf, die unter Ausbildung der Schwenkwachse mit einer komplementären Schwenklagerfläche des ersten Maulteils gleitbeweglich zusammenwirkt. Hierdurch kann auf ein gesondertes Bauteil zum Ausbilden einer um die Schwenkachse schwenkbeweglichen Stift- oder Bolzenverbindung zwischen dem ersten Maulteil und dem zweiten Maulteil verzichtet werden. Die reduzierte Bauteilanzahl ermöglicht einen vereinfachten Aufbau und damit einhergehend eine vorteilhafte Herstellung der Werkzeugeinrichtung und/oder des chirurgischen Instruments. Die Schwenklagerfläche des zweiten Maulteils und die komplementäre Schwenklagerfläche des ersten Maulteils wirken um die Schwenkachse gleitbeweglich und radial zur Schwenkachse wenigstens einseitig, vorzugsweise radial nach innen, formschlüssig zusammen. Die Schwenklagerfläche des zweiten Maulteils ist vorzugsweise konvex und die komplementäre Schwenklagerfläche des ersten Maulteils ist vorzugsweise komplementär konkav oder umgekehrt.

In weiterer Ausgestaltung der Erfindung ist die Schwenklagerfläche des zweiten Maulteils durch einen Außenumfang eines Querstegabschnitts gebildet, welcher die Aufnahmeaussparung in Axialrichtung des Steuerstifts übergreift. Der Querstegabschnitt stellt eine stoffschlüssige und/oder einstückige Verbindung zwischen in Axialrichtung des Steuerstifts gegenüberliegenden Innenwandungen der Aufnahmeaussparung her. Der Querstegabschnitt kann auch als Brücke bezeichnet werden. Der Querstegabschnitt ist parallel zur Schwenkachse längserstreckt. Die Schwenklagerfläche des zweiten Maulteils ist vorzugsweise durch eine Unterseite des Querstegabschnitts oder alternativ durch dessen Oberseite gebildet.

In weiterer Ausgestaltung der Erfindung weist das zweite Maulteil eine Schwenkführungsfläche auf, welche gekrümmt und konzentrisch zu der Schwenkachse längserstreckt ist, und welche mit einer komplementären Schwenkführungsfläche des ersten Maulteils gleitbeweglich zusammenwirkt. Die Schwenkführungsfläche des ersten Maulteils und die komplementäre Schwenkführungsfläche des zweiten Maulteils wirken um die Schwenkachse gleitbeweglich und radial zu dieser formschlüssig zusammen. Die Schwenkführungsfläche und die komplementäre Schwenkführungsfläche dienen einer verbesserten Führung der Schwenkbeweglichkeit des Werkzeugmauls um die Schwenkachse. Sofern die Schwenkachse in einem in Bezug auf eine Hochrichtung oberen Bereich des zweiten Maulteils angeordnet ist, ist die Schwenkführungsfläche vorzugsweise in einem unteren Bereich des zweiten Maulteils angeordnet oder umgekehrt. Durch die zwischen der Schwenkführungsfläche und der komplementären Schwenkführungsfläche ausgebildete Schwenkführung kann auf gesonderte diesbezügliche Bauteile verzichtet und ein nochmals vereinfachter Aufbau erreicht werden. Mit anderen Worten ist die Schwenkführung unmittelbar zwischen dem ersten Maulteil und dem zweiten Maulteil ausgebildet.

In weiterer Ausgestaltung der Erfindung ist ein an dem zweiten Maulteil angeordneter und/oder ausgebildeter Anschlagabschnitt vorgesehen, welcher in einer Öffnungsstellung des Werkzeugmauls mit einem komplementären Anschlagabschnitt des ersten Maulteils um die Schwenkachse formschlüssig zusammenwirkt. Hierdurch wird einem über die Öffnungsstellung hinausgehenden Öffnen des Werkzeugmauls entgegengewirkt. Der komplementäre Anschlagabschnitt des ersten Maulteils ist vorzugsweise proximal an dessen Steuerabschnitt angeordnet. Der dem zweiten Maulteil zugeordnete Anschlagabschnitt ist vorzugsweise im Bereich der Aufnahmeaussparung angeordnet und/oder ausgebildet. Besonders bevorzugt ist der Anschlagabschnitt ein Wandabschnitt einer Verbindungshülse, die auf ein proximales Ende des zweiten Maulteils aufschiebbar ist.

In weiterer Ausgestaltung der Erfindung ist das zweite Maulteil in Bezug auf die Schwenkachse feststehend, und das erste Maulteil rotiert beim Öffnen und Schließen des Werkzeugmauls relativ zu dem ersten Maulteil um die Schwenkachse. In diesem Zusammenhang kann auch von einer unilateralen Schwenkbeweglichkeit des Werkzeugmauls gesprochen werden. Dies im Unterschied zu einer bilateralen Schwenkbeweglichkeit, bei welcher beide Maulteile zum Öffnen und Schließen des Werkzeugmauls jeweils um die Schwenkachse rotieren. Die Ausgestaltung mit unilateraler Schwenkbeweglichkeit gestattet einen weiter vereinfachten Aufbau der Werkzeugeinrichtung.

In weiterer Ausgestaltung der Erfindung weist das zweite Maulteil einen einstückigen Gehäuseabschnitt und einen distal an dem Gehäuseabschnitt montierten Wirkabschnitt auf, wobei der Gehäuseabschnitt wenigstens die Aufnahmeaussparung mitsamt des wenigstens einen Steuervorsprungs und/oder die Schwenklagerfläche aufweist. Der Wirkabschnitt ist vorzugsweise formschlüssig an dem Gehäuseabschnitt montiert. Der Wirkabschnitt kann starr oder um eine, vorzugsweise parallel zu der Schwenkachse erstreckte, Kippachse kippbeweglich an dem Gehäuseabschnitt festgelegt sein. Sofern als solche vorgesehen, sind vorzugsweise auch die Schwenklagerfläche, die Schwenkführungsfläche, die Längsführungsfläche und/oder der Anschlagabschnitt an dem Gehäuseabschnitt ausgebildet. Durch eine solche Integralbauweise werden vielfältige fertigungs- und montageseitige Vorteile erreicht.

In weiterer Ausgestaltung der Erfindung ist die Steuernut zwischen einem proximalen Nutende und einem distalen Nutende durchgängig gerade längserstreckt. Im Vergleich zu einer lediglich abschnittsweise geraden, abgewinkelten Längserstreckung der Steuernut kann hierdurch eine nochmals vereinfachte Fertigung erreicht werden.

Die Erfindung betrifft zudem ein chirurgisches Instrument mit den Merkmalen des Anspruchs 10. Das erfindungsgemäße chirurgische Instrument weist einen entlang einer Schaftlängsachse längserstreckten Schaft, eine distal an dem Schaft angeordnete Werkzeugeinrichtung gemäß der vorhergehenden Beschreibung, und eine proximal an dem Schaft angeordnete Griffeinrichtung mit einem Bedienelement auf, welches über ein Zug-Druck-Element mit der Werkzeugeinrichtung wirkverbunden ist, wobei das Zug-Druck-Element mittels einer Bedienung des Bedienelements entlang einer Längsbewegungsachse translatorisch verlagerbar ist, wobei der Steuerstift der Werkzeugeinrichtung mittels einer Längsbewegung des Zug-Druck-Elements zwischen einer proximalen Stiftendlage und einer distalen Stiftendlage entlang der Steuernut gleitbeweglich verlagerbar ist und hierbei zum Öffnen und/oder Schließen des Werkzeugmauls ein um die Schwenkachse wirkendes Drehmoment auf das erste Maulteil bewirkt.

In weiterer Ausgestaltung der Erfindung weist das zweite Maulteil eine Längsführungsfläche auf, welche parallel zu der Längsbewegungsachse des Zug-Druck-Elements längserstreckt ist, und an welcher eine komplementäre Längsführungsfläche des Zug-Druck-Elements bei dessen Längsbewegung wenigstens zeitweise gleitbeweglich geführt ist. Die Längsführung zwischen dem zweiten Maulteil und dem Zug-Druck-Element wirkt einer ungewollten quer zur Längsbewegungsachse gerichteten Bewegung des Zug-Druck-Elements entgegen. Gleichzeitig kann hierdurch eine ungewollte Verlagerung des an dem Zug-Druck-Element angreifenden Steuerstifts vermieden werden. Dies unterstützt eine bestimmungsgemäße Verlagerung des Steuerstifts entlang des wenigstens einen Steuervorsprungs. Insbesondere kann einem ungewollten Abheben des Steuerstifts von der durch den Steuervorsprung gebildeten Steuerfläche entgegengewirkt werden. Die Längsführungsfläche ist vorzugsweise in einem proximalen Bereich des zweiten Maulteils angeordnet. Die komplementäre Längsführungsfläche ist vorzugsweise durch eine Ober- oder Unterseite des Zug-Druck-Elements ausgebildet. Die gleitbewegliche Führung zwischen dem zweiten Maulteil und dem Zug-Druck-Element ist vorzugsweise lediglich zeitweise ausgebildet, beispielsweise dann, wenn der Steuerstift sich im Bereich seiner proximalen Stiftendlage bewegt.

In weiterer Ausgestaltung der Erfindung weist die Steuerfläche einen proximalen Flächenabschnitt und einen distalen Flächenabschnitt auf, welche zu der Längsbewegungsachse derart unterschiedlich geneigt längserstreckt sind, dass bei der Verlagerung des Steuerstifts zwischen der proximalen und der distalen Stiftendlage unterschiedliche Übersetzungsverhältnisse zwischen der Längsbewegung des Zug-Druck-Elements und der Schwenkbewegung des Werkzeugmauls erreicht sind. Entsprechend unterschiedliche Übersetzungsverhältnisse ergeben sich zwangsläufig für die mit der Längsbewegung einhergehende Längskraft und die mit der Schwenkbewegung des Werkzeugmauls einhergehende, auf zwischen den Maulteilen befindliches Körpergewebe ausübbare Klemmkraft. Durch die unterschiedlich geneigt längserstreckten Flächenabschnitte der Steuerfläche kann ausgehend von der Öffnungsstellung des Werkzeugmauls zunächst ein vergleichsweise schnelles und hiernach ein vergleichsweise langsames Schließen des Werkzeugmauls erreicht werden. Hierdurch kann ein initiales Öffnen des Werkzeugmauls mit einer vergleichsweise geringen Längsbewegung des Zug-Druck-Elements und damit mit einem geringen manuellen Bedienweg des Bedienelements erreicht werden. Mit anderen Worten kann ein am Bedienelement erforderlicher Hubweg reduziert werden, was insbesondere für kleinere Hände vorteilhaft ist. Gleichzeitig kann mit einer vergleichsweise geringen manuellen Bedienkraft am Bedienelement eine vergleichsweise hohe Klemmkraft am Werkzeugmaul erzeugt werden. Eine im Sinne dieser Ausgestaltung variable Übersetzung kann auch mittels eines nicht-linearen, d.h. über seiner Länge unterschiedlich geneigten, Langloches erreicht werden.

In weiterer Ausgestaltung der Erfindung ist der proximale Flächenabschnitt parallel und/oder wenigstens im Wesentlichen parallel zu der Längsbewegungsachse längserstreckt. Bewegt sich der Steuerstift entlang des proximalen Flächenabschnitts, bewirkt dies bei dieser Ausgestaltung der Erfindung ein vergleichsweise kleines Übersetzungsverhältnis. Demnach bewirkt eine vergleichsweise geringe manuelle Bedienkraft am Bedienelement eine vergleichsweise hohe Klemmkraft am Werkzeugmaul. Vorzugsweise ist der Steuerstift in seiner proximalen Stiftendlage im Bereich des proximalen Flächenabschnitts positioniert.

In weiterer Ausgestaltung der Erfindung ist der distale Flächenabschnitt gekrümmt und konzentrisch zu der Schwenkachse längserstreckt. Je nach Bewegungsrichtung des Steuerstifts werden hierdurch unterschiedliche Vorteile erreicht. Bei einer Verlagerung in Richtung der proximalen Stiftendlage weist der Steuerstift bei seiner Bewegung entlang des distalen Flächenabschnitts einen stets gleichbleibenden Hebelarm in Bezug auf die Schwenkachse auf. Hierdurch wird im Bereich des distalen Flächenabschnitts ein gleichbleibendes Übersetzungsverhältnis gewährleistet. Dies kann Vorteile im Hinblick auf eine Anwendungs- und Bedienfreundlichkeit bieten. Bei einer Bewegung in Richtung der distalen Stiftendlage wird durch die gekrümmte und konzentrische Längserstreckung des distalen Flächenabschnitts vorzugsweise ein über der Schwenkbewegung konstantes Radialspiel zwischen dem Steuerstift und dem distalen Flächenabschnitt gewährleistet. Bei einer durch den Bediener eingeleiteten Bewegungsumkehr bietet dies zusätzliche Vorteile, da der Bediener stets mit einem gleichbleibenden Spiel und somit einen konstanten Leerhub am Bedienelement rechnen kann. Hierdurch wird die Anwendungs- und Bedienfreundlichkeit weiter verbessert.

In weiterer Ausgestaltung der Erfindung ist der distale Flächenabschnitt gerade und tangential zu einem konzentrisch zu der Schwenkachse angeordneten Kreisbogen längserstreckt. Im Vergleich zu der vorhergehenden Ausgestaltung der Erfindung kann eine ähnliche Anwendungs- und Bedienfreundlichkeit bei gleichzeitig vereinfachter Fertigbarkeit des distalen Flächenabschnitts erreicht werden.

In weiterer Ausgestaltung der Erfindung ist der Steuerstift bei einer Verlagerung in Richtung der distalen Stiftendlage wenigstens zeitweise an dem distalen Nutende der Steuernut gehalten und rotiert gemeinsam mit dem ersten Maulteil um die Schwenkachse. Hierdurch kann insbesondere einem übermäßigen Radialspiel zwischen dem Steuerstift und der Steuerfläche des Steuervorsprungs, insbesondere des distalen Flächenabschnitts der Steuerfläche, entgegengewirkt werden.

Die Erfindung betrifft zudem ein Verfahren zur Herstellung einer Werkzeugeinrichtung gemäß der vorhergehenden Beschreibung. Das erfindungsgemäße Verfahren weist den Schritt auf: Urformen des zweiten Maulteils, wobei wenigstens ein den wenigstens einen Steuervorsprung tragender Gehäuseabschnitt des zweiten Maulteils urgeformt wird. Das Urformen erfolgt vorzugsweise mittels Pulverspritzgießens (Metal Injection Molding). Durch die erfindungsgemäße Gestaltung des zweiten Maulteils kann ein vergleichsweise einfach gestaltetes Urformwerkzeug verwendet werden. Insbesondere kann auf Schieber, Stempel oder dergleichen an dem Urformwerkzeug verzichtet werden.

In weiterer Ausgestaltung der Erfindung weist das Verfahren den Schritt auf: formschlüssiges Zusammenfügen des ersten Maulteils und des urgeformten Gehäuseabschnitts, wobei eine an dem Gehäuseabschnitt ausgebildete Schwenklagerfläche des zweiten Maulteils und eine komplementäre Schwenklagerfläche des ersten Maulteils unter Ausbildung der Schwenkachse um dieselbe gleitbeweglich und radial zu der Schwenkachse formschlüssig aneinander festgelegt werden. Dementsprechend werden das erste Maulteil und das zweite Maulteil zur Ausbildung der Schwenkbeweglichkeit des Werkzeugmauls nicht etwa mit einem gesonderten Stift-, Achs- oder Bolzenelement miteinander verbunden. Vielmehr werden das erste Maulteil und das zweite Maulteil unmittelbar um die Schwenkachse gleitbeweglich und radial zu derselben formschlüssig zusammengefügt. Die Fügeverbindung wird zwischen der Schwenklagerfläche des zweiten Maulteils und der komplementären Schwenklagerfläche des ersten Maulteils ausgebildet.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt eine schematische Seitenansicht einer Ausführungsform eines erfindungsgemäßen chirurgischen Instruments mit einer Griffeinrichtung, einem längserstreckten Schaft und einer Ausführungsform einer erfindungsgemäßen Werkzeugeinrichtung,
- Fig. 2: eine perspektivische Explosionsdarstellung der Werkzeugeinrichtung und weiterer Bauteile des chirurgischen Instruments, wobei die Werkzeugeinrichtung ein erstes Maulteil und ein mehrstückiges zweites Maulteil aufweist,
- Fig. 3: ein dem zweiten Maulteil zugeordneter Gehäuseabschnitt entlang eines Schnitts III-III nach Fig. 4,
- Fig. 4: den Gehäuseabschnitt nach Fig. 3 in einer Draufsicht,
- Fig. 5: eine Variante des Gehäuseabschnitts nach den Fig. 3 und 4,
- Fig. 6: das erste Maulteil in einer schematischen Seitenansicht,
- Fig. 7: das erste Maulteil nach Fig. 6 in einer schematischen Draufsicht,
- Fig. 8: die Werkzeugeinrichtung in einer schematischen Draufsicht und unter Einzeichnung eines für die Fig. 9 bis 13 maßgeblichen Stufenschnitts IX-IX,
- Fig. 9: eine Schnittdarstellung der Werkzeugeinrichtung, wobei ein durch das erste und zweite Maulteil gebildetes Werkzeugmaul eine Öffnungsstellung einnimmt,
- Fig. 10: eine weitere Schnittdarstellung der Werkzeugeinrichtung, wobei das Werkzeugmaul ausgehend von der Öffnungsstellung in Richtung einer Schließstellung verlagert ist,
- Fig. 11: eine weitere Schnittdarstellung der Werkzeugeinrichtung, wobei das Werkzeugmaul die Schließstellung einnimmt,
- Fig. 12: eine weitere Schnittdarstellung der Werkzeugeinrichtung mit geschlossenem Werkzeugmaul und bei einer Verlagerung in Richtung der Öffnungsstellung,
- Fig. 13: eine weitere Schnittdarstellung in einer weitergehend in Richtung der Öffnungsstellung verlagerten Funktionsstellung,
- Fig. 14: ein schematisches Diagramm, in welchem ein Öffnungswinkel des Werkzeugmauls über einem Bedienweg eines Bedienelements der Griffeinrichtung aufgetragen ist,
- Fig. 15: eine schematische Darstellung zur Verdeutlichung eines Verfahrensschritts zur Herstellung der Werkzeugeinrichtung,
- Fig. 16: eine weitere schematische Darstellung zur Verdeutlichung eines weiteren Verfahrensschritts,
- Fig. 17, 18: weitere schematische Darstellungen zur Verdeutlichung eines weiteren Verfahrensschritts,
- Fig. 19: eine weitere schematische Darstellung zur Verdeutlichung eines weiteren Verfahrensschritts in Bezug auf eine winkelstarre Montage der Werkzeugeinrichtung an dem Schaft und
- Fig. 20, 21: weitere schematische Darstellungen zur Verdeutlichung eines weiteren Verfahrensschritts in Bezug auf eine knickbewegliche Montage der Werkzeugeinrichtung an dem Schaft.

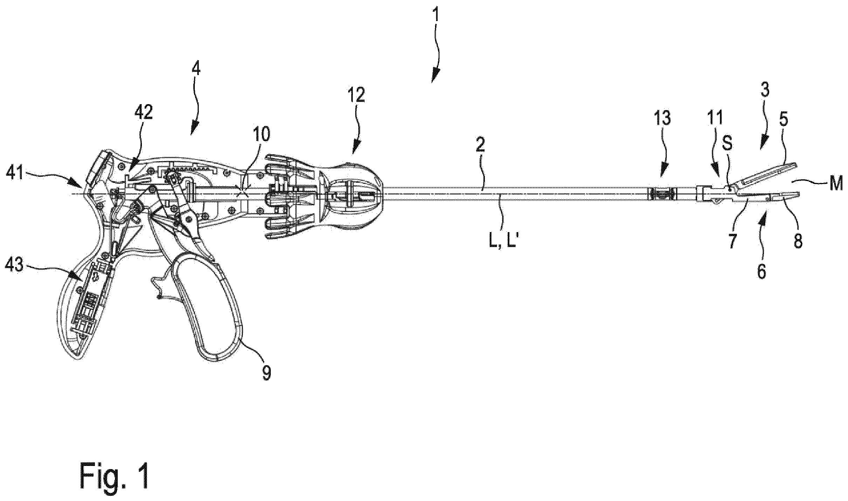

Gemäß Fig. 1 weist ein chirurgisches Instrument 1 einen entlang einer Schaftlängsachse L längserstreckten Schaft 2, eine distal an dem Schaft 2 angeordnete Werkzeugeinrichtung 3 und eine proximal an dem Schaft 2 angeordnete Griffeinrichtung 4 auf.

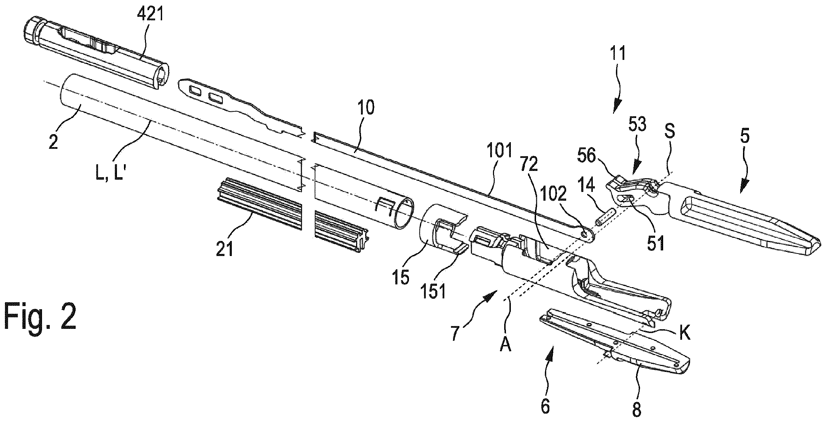

Die Werkzeugeinrichtung 3 weist ein erstes Maulteil 5 und ein zweites Maulteil 6 auf. Die beiden Maulteile 5, 6 sind unter Ausbildung eines öffenbaren und schließbaren Werkzeugmauls M um eine quer zur Schaftlängsachse L orientierte Schwenkachse S (Fig. 2) relativ zueinander schwenkbeweglich.

Das zweite Maulteil 6 ist bei der gezeigten Ausführungsform mehrstückig gestaltet und weist einen proximalen Gehäuseabschnitt 7 und einen distalen Wirkabschnitt 8 auf, der auf noch näher beschriebene Weise formschlüssig an einem distalen Ende des Gehäuseabschnitts 7 festgelegt ist. Eine solche mehrstückige Gestaltung des zweiten Maulteils 6 ist vorteilhaft, aber im Hinblick auf die erfindungsgemäße Lehre als nicht wesentlich zu erachten. Dementsprechend sieht eine nicht gezeigte Ausführungsform eine einstückige Gestaltung des zweiten Maulteils vor.

Die Griffeinrichtung 4 weist ein manuell bewegliches Bedienelement 9 auf, das über ein Zug-Druck-Element 10 mit der Werkzeugeinrichtung 3 wirkverbunden ist. Hierdurch kann das Werkzeugmaul M mittels einer Bedienung des Bedienelements 9 zum Fassen und Klemmen von zwischen den Maulteilen 5, 6 befindlichem Körpergewebe geöffnet und/oder geschlossen werden.

Die anhand Fig. 1 gezeigte Gestaltung der Griffeinrichtung 4 ist als rein exemplarisch zu erachten. Vorliegend weist die Griffeinrichtung 4 ein aus mehreren nicht näher bezeichneten Gehäuseteilen gebildetes Gehäuse auf, an und/oder in welchem weitere Komponenten der Griffeinrichtung, insbesondere das Bedienelement 9, gelagert sind. Anhand Fig. 1 ist lediglich eine nicht näher bezeichnete Gehäusehälfte des besagten Gehäuses gezeigt. Zur beweglichen Lagerung des Bedienelements 9 an dem Gehäuse weist die Griffeinrichtung 4 eine Bedienmechanik 41 auf, deren Gestaltung grundsätzlich bekannt und beispielsweise in der deutschen Patentanmeldung DE 10 2017 109 891 A1 beschrieben ist. Zudem ist eine Koppelmechanik 42 vorgesehen, die ebenfalls in der besagten Patentanmeldung beschrieben ist und einer kraft- und bewegungsübertragenden Ankopplung des Bedienelements 9 an das Zug-Druck-Element 10 dient. Das Bedienelement 9 ist relativ zu dem Gehäuse der Griffeinrichtung 4 zwischen einer distalen Endlage (Fig. 1) und einer zeichnerisch nicht näher dargestellten proximalen Endlage manuell hubbeweglich. In der proximalen Endlage ist das Bedienelement 9 auf grundsätzlich bekannte Weise mittels einer Arretiermechanik 43, die auch als Umlaufsperre bezeichnet wird, arretierbar. Zur Unterstützung einer in Richtung der distalen Endlage des Bedienelements 9 gerichteten Bewegung kann die Bedienmechanik 41 ein nicht näher bezeichnetes Federelement aufweisen.

Das Zug-Druck-Element 10 ist mittels der besagten Hubbewegung des Bedienelements 9 entlang einer Längsbewegungsachse L' translatorisch verlagerbar. Die Längsbewegungsachse L' des Zug-Druck-Elements 10 fällt bei der gezeigten Ausführungsform mit der Schaftlängsachse L zusammen. Das Zug-Druck-Element 10 ist an seinem distalen Ende auf noch näher beschriebene Weise mit einer Steueranordnung 11 der Werkzeugeinrichtung 3 wirkverbunden. Die Steueranordnung 11 ist zur Umformung der transtatorischen Bewegung des Zug-Druck-Elements 10 in eine um die Schwenkachse S gerichtete Schwenkbewegung des ersten Maulteils 5 und/oder des zweiten Maulteils 6 eingerichtet.

Bei der gezeigten Ausführungsform ist das zweite Maulteil 6 in Bezug auf die Schwenkachse S feststehend und das erste Maulteil 5 ist in Bezug auf die Schwenkachse S schwenkbeweglich. Insoweit wird zum Öffnen und Schließen des Werkzeugmauls M lediglich das erste Maulteil 5 verschwenkt. In diesem Zusammenhang kann auch von einer unilateralen Schwenkbeweglichkeit gesprochen werden. Dies im Unterschied zu einer bilateralen Schwenkbeweglichkeit, bei welcher beide Maulteile relativ zu der Schwenkachse S schwenkbeweglich sind. Bei einer zeichnerisch nicht dargestellten Ausführungsform kann eine solche bilaterale Schwenkbeweglichkeit vorgesehen sein.

Bei der anhand Fig. 1 gezeigten Ausführungsform ist die gesamte Werkzeugeinrichtung 3 relativ zu der Schaftlängsachse L abwinkelbar an dem Schaft 2 festgelegt. Zum Abwinkeln der Werkzeugeinrichtung 3 ist eine Abwinkelungsmechanik 12, 13 mit einer griffseitigen Bedieneinheit 12 und einer schaftseitigen Wirbeleinheit 13 vorgesehen. Der Aufbau und die Funktionsweise der Abwinkelungsmechanik 12, 13 sind grundsätzlich bekannt und beispielsweise in dem europäischen Patent EP 2 688 501 B1 beschrieben. Alternativ kann die Werkzeugeinrichtung 3 starr und/oder lediglich um die Schaftlängsachse L rotierbar an dem Schaft 2 festgelegt sein.

Bei der vorliegenden Ausführungsform ist das Zug-Druck-Element 10 bandförmig gestaltet und innerhalb des Schafts 2 längserstreckt (Fig. 2). Insbesondere zur verbesserten Längsführung des Zug-Druck-Elements 10 innerhalb des Schafts 2 ist vorliegend ein Führungselement 21 in dem Schaft angeordnet.

Das Zug-Druck-Element 10 wirkt an seinem proximalen Ende mit einem Bauteil 421 der Koppelmechanik 42 zusammen. Das Zug-Druck-Element 10 wirkt an seinem distalen Ende mit einem Steuerstift 14 der Steueranordnung 11 zusammen.

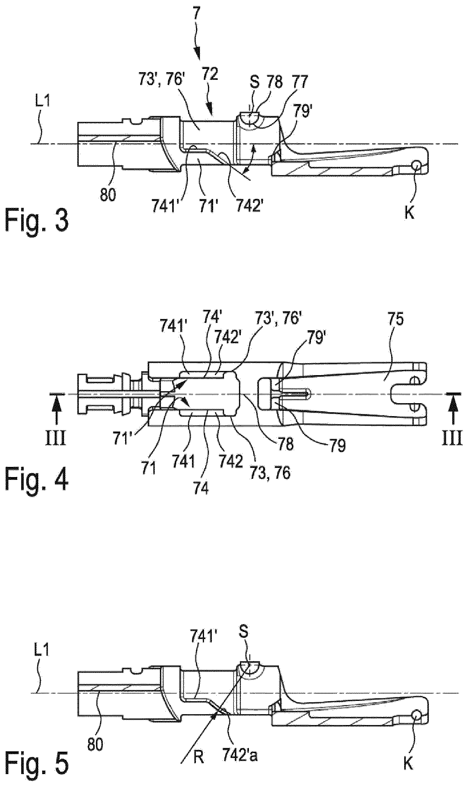

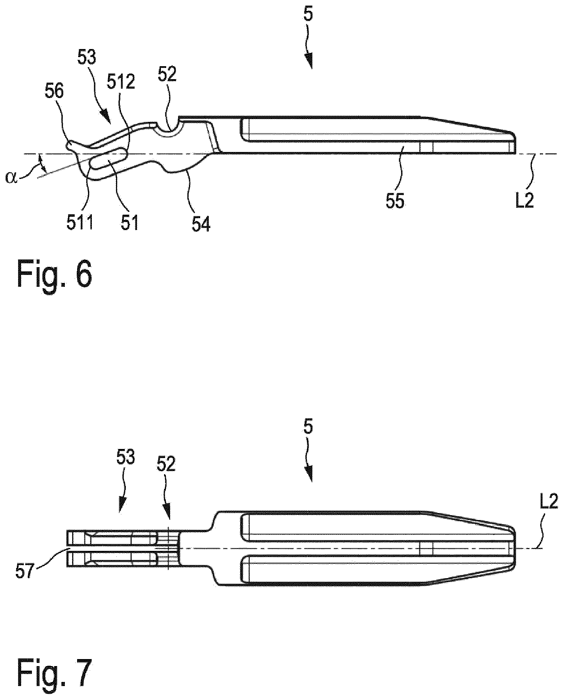

Die Steueranordnung 11 weist wenigstens eine Steuernut 51 (Fig. 6) und wenigstens einen Steuervorsprung 71 auf. Die wenigstens eine Steuernut 51 ist dem ersten Maulteil 5 zugeordnet. Der wenigstens eine Steuervorsprung 71 ist dem zweiten Maulteil 6 zugeordnet und vorliegend an dessen Gehäuseabschnitt 7 ausgebildet. Der Steuerstift 14 wirkt bei einer translatorischen Verlagerung des Zug-Druck-Elements 10 gleitbeweglich mit der Steuernut 51 und dem Steuervorsprung 71 zusammen. Bevor auf die weitere Funktionsweise der Steueranordnung 11 bei einem Öffnen und Schließen des Werkzeugmauls 12 eingegangen wird, werden zunächst weitere räumlich-körperliche Merkmale des ersten und zweiten Maulteils 5, 6 anhand der Fig. 3 bis 7 erläutert.

Wie insbesondere anhand der Fig. 3 und 4 gezeigt ist, weist das zweite Maulteil 6, vorliegend an seinem Gehäuseabschnitt 7, eine Aufnahmeaussparung 72 auf. Der wenigstens eine Steuervorsprung 71 ragt in Axialrichtung A (Fig. 2) des Steuerstifts 14 nach innen von einer Innenwandung 73 der Aufnahmeaussparung 72 ab. Dabei bildet der wenigstens eine Steuervorsprung 71 eine Steuerfläche 74 der Steueranordnung 11, die auf noch näher beschriebene Weise mit dem Steuerstift 14 zusammenwirkt. Bei der gezeigten Ausführungsform ist die Steuerfläche 74 auf noch näher beschriebene Weise abschnittsweise mit unterschiedlichen Neigungswinkeln in Relation zu der Schaftlängsachse L und/oder der Längsbewegungsachse L' längserstreckt. Bei einer zeichnerisch nicht dargestellten Ausführungsform kann die Steuerfläche stattdessen durchgängig parallel oder geneigt zu den besagten Längsachsen längserstreckt sein.

Das zweite Maulteil 6, insbesondere dessen Gehäuseabschnitt 7, weist vorliegend einen spiegelsymmetrischen Aufbau auf (Fig. 4). Dementsprechend ist ein weiterer Steuervorsprung 71' vorgesehen, der in Axialrichtung A des Steuerstifts 14 nach innen von einer Innenwandung 73' der Aussparung 72 abragt. Der weitere Steuervorsprung 71' bildet eine weitere Steuerfläche 74' aus. Der Steuerstift 14 wirkt bei seiner Bewegung gleitbeweglich mit beiden Steuervorsprüngen 71, 71' und den an diesen ausgebildeten Steuerflächen 74, 74' zusammen. Zur Vermeidung von Wiederholungen wird in erster Linie auf den Steuervorsprung 71 und dessen Steuerfläche 74 im Detail Bezug genommen. Das diesbezügliche Offenbarte gilt sinngemäß auch im Hinblick auf den weiteren Steuervorsprung 71' und dessen Steuerfläche 74'. Umgekehrt gilt dasselbe.

Der Gehäuseabschnitt 7 ist entlang seiner Längsachse L1 zwischen einem proximalen Ende und einem distalen Ende längserstreckt. Die Aufnahmeaussparung 72 ist bei der gezeigten Ausführungsform in etwa mittig zwischen dem proximalen und distalen Ende des Gehäuseabschnitts 7 angeordnet und in Bezug auf die Längsachse L1 in distaler Richtung sowie - in Bezug auf die Zeichenebene der Fig. 3 - nach oben offen. Das proximale Ende des Gehäuseabschnitts 7 ist in betriebsfertigem Zustand formschlüssig am distalen Ende des Schafts 2 festgelegt (Fig. 2). Der Wirkabschnitt 8 ist am proximalen Ende des Gehäuseabschnitts 7 angeordnet und um eine Kippachse K (Fig. 2) kippbeweglich sowie entlang der Längsachse L1 formschlüssig an einem Aufnahmeabschnitt 75 des Gehäuseabschnitts 7 montiert. Der Wirkabschnitt 8 ist zum Einwirken auf das Körpergewebe vorgesehen und bei einer zeichnerisch nicht dargestellten Ausführungsform starr an dem Gehäuseabschnitt 7 montiert. Bei einer weiteren Ausführungsform kann der Wirkabschnitt 8 einstückig mit dem Gehäuseabschnitt 7 ausgebildet sein.

In betriebsfertig montiertem Zustand ist der Steuerstift 14 innerhalb der Aufnahmeaussparung 72 angeordnet. Hierbei ist der Steuerstift 14 zwischen den Innenwandungen 73, 73' in Axialrichtung A formschlüssig gehalten. Die Innenwandungen 73, 73' bilden hierbei gleichsam Sicherungsflächen 76, 76' zur Axialsicherung des Steuerstifts 14. Die beiden Innenwandungen 73, 73' und damit die beiden Sicherungsflächen 76, 76' sind in Axialrichtung A um einen Abstand voneinander beabstandet, der geringfügig größer ist als eine axiale Länge des Steuerstifts 14.

Vorliegend weist das zweite Maulteil 6, genauer dessen Gehäuseabschnitt 7, eine Schwenklagerfläche 77 auf, die unter Ausbildung der Schwenkachse S mit einer komplementären Schwenklagerfläche 52 des ersten Maulteils 5 zusammenwirkt (Fig. 6). Die Schwenklagerfläche 77 ist vorliegend durch einen Außenumfang eines Querstegabschnitts 78 gebildet, der die Aufnahmeaussparung 72 in Axialrichtung A des Steuerstifts 14 übergreift. Der Querstegabschnitt 78 kann auch als Brücke bezeichnet werden. Die Schwenklagerfläche 77 ist durch eine - in Bezug auf die Zeichenebene der Fig. 3 - Unterseite des Querstegabschnitts 78 ausgebildet. Die Schwenkachse S ist oberhalb der Steuerfläche 74 angeordnet. Im betriebsfertig montierten Zustand untergreift ein die Steuernut 51 tragender Steuerabschnitt 53 des ersten Maulteils (Fig. 6) den Querstegabschnitt 78, wobei die komplementäre Schwenklagerfläche 52 um die Schwenkachse S schwenkbeweglich und in radialer Richtung nach innen formschlüssig an der Schwenklagerfläche 77 abgestützt ist.

Weiter weist das zweite Maulteil 6, genauer: dessen Gehäuseabschnitt 7, vorliegend eine Schwenkführungsfläche 79 auf. Die Schwenkführungsfläche 79 ist vorliegend an einem distalen Ende der Gehäuseaussparung 72 angeordnet. Dabei ist die Schwenkführungsfläche 79 gekrümmt und konzentrisch zu der Schwenkachse S längserstreckt. Im betriebsfertig montierten Zustand wirkt die Schwenkführungsfläche 79 um die Schwenkachse S gleitbeweglich und radial zur Schwenkachse S nach außen formschlüssig mit einer komplementären Schwenkführungsfläche 54 des ersten Maulteils zusammen (Fig. 6). Vorliegend ist eine weitere Schwenkführungsfläche 79' vorgesehen. Die beiden Schwenkführungsflächen 79, 79' des Gehäuseabschnitts 7 sind in Bezug auf dessen Längsachse L1 spiegelsymmetrisch angeordnet und/oder gestaltet. Die weitere Schwenkführungsfläche 79' wirkt in entsprechender Weise mit einer nicht näher ersichtlichen weiteren komplementären Schwenkführungsfläche des ersten Maulteils 5 zusammen. Bei einer zeichnerisch nicht dargestellten Ausführungsform sind lediglich eine Schwenkführungsfläche und eine komplementäre Schwenkführungsfläche vorgesehen.

Vorliegend weist das zweite Maulteil 6 an seinem Gehäuseabschnitt 7 eine Längsführungsfläche 80 auf. Die Längsführungsfläche 80 ist parallel zu der Längsbewegungsachse L' des Zug-Druck-Elements 10 längserstreckt und dient einer einseitig vertikalen Abstützung des Zug-Druck-Elements 10. Hierfür wirkt die Längsführungsfläche 80 entlang der Längsbewegungsachse L' gleitbeweglich und in einer senkrecht zur Längsbewegungsachse L' ausgerichteten Hochrichtung einseitig formschlüssig mit einer komplementären Längsführungsfläche 101 des Zug-Druck-Elements 10 zusammen. Die komplementäre Längsführungsfläche 101 ist vorliegend durch eine obere Flachseite des Zug-Druck-Elements gebildet. Die gleitbewegliche Abstützung des Zug-Druck-Elements 10 erfolgt vorliegend lediglich zeitweise und in Abhängigkeit eines jeweiligen Verlagerungszustands des Steuerstifts 14 entlang der Steuerfläche 74. Hierauf wird nachfolgend noch näher eingegangen. Die Längsführungsfläche 80 ist am proximalen Ende des Gehäuseabschnitts 7 angeordnet.

Bei der gezeigten Ausführungsform weist die Steuerfläche 74 einen proximalen Flächenabschnitt 741 und einen distalen Flächenabschnitt 742 auf. Die beiden Flächenabschnitte 741, 742 sind relativ zu der Längsachse L1 und damit auch zu der zu dieser parallelen ausgerichteten Schaftlängsachse L und/oder Längsbewegungsachse L' unterschiedlich geneigt längserstreckt. Der proximale Flächenabschnitt 741 ist parallel zur Längsbewegungsachse L' längserstreckt. Der distale Flächenabschnitt 742 ist dementgegen geneigt längserstreckt. Die Neigung des distalen Flächenabschnitts 742 verläuft in distaler Richtung von oben nach unten (Fig. 3). Der distale Flächenabschnitt 742 ist gerade längserstreckt und tangential zu einem konzentrisch zu der Schwenkachse S angeordneten Kreisbogen ausgerichtet.

Anhand Fig. 5 ist eine Variante mit unterschiedlich gestaltetem distalem Flächenabschnitt 742a gezeigt, wobei Fig. 5 lediglich die symmetrisch gegenüberliegenden Flächenabschnitte 741' und 742'a abbildet. Im Unterschied zu dem distalen Flächenabschnitt 742 der Variante nach den Fig. 3 und 4 ist der distale Flächenabschnitt 742a gekrümmt und konzentrisch zu der Schwenkachse S längserstreckt. Abgesehen von der Gestaltung des distalen Flächenabschnitts 742a ist die anhand Fig. 5 gezeigte Variante identisch zu der anhand der Fig. 3 und 4 gezeigten Ausführungsform. Der distale Flächenabschnitt 742a ist vorliegend um einen Radius R von der Schwenkachse S beabstandet.

Im Vergleich zu dem gekrümmt längserstreckten distalen Flächenabschnitt 742a ist der gerade längserstreckte distale Flächenabschnitt 742 vergleichsweise einfach zu fertigen. Demgegenüber bietet die gekrümmte Gestaltung des distalen Flächenabschnitts 742a noch näher erläuterte Vorteile insbesondere im Hinblick auf eine möglichst spielfreie Führung des Steuerstifts 14.

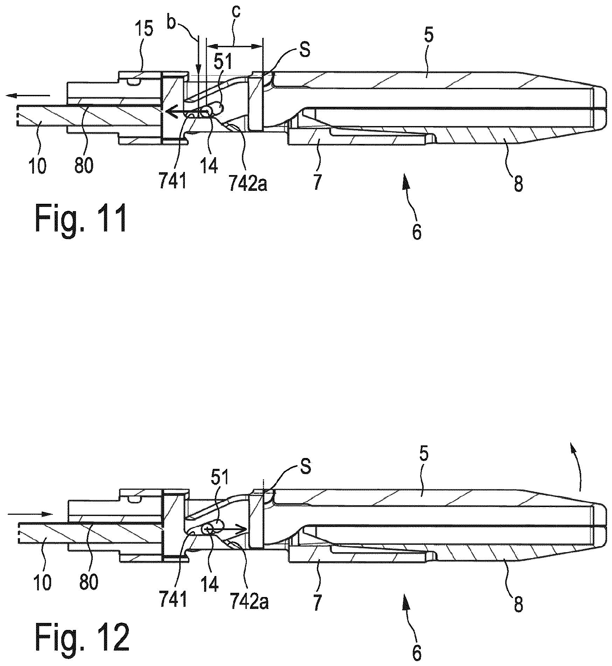

Das erste Maulteil 5 ist entlang einer Längsachse L2 zwischen einem distalen Ende und einem proximalen Ende längserstreckt. Der die Steuernut 51 tragende Steuerabschnitt 53 ist proximal angeordnet. Das erste Maulteil 5 weist, ähnlich wie das zweite Maulteil 6, einen distal angeordneten Wirkabschnitt 55 auf. Im Unterschied zu dem Wirkabschnitt 8 des zweiten Maulteils 6 ist der Wirkabschnitt 55 einstückig mit den übrigen Abschnitten des ersten Maulteils 5 zusammenhängend. Mit anderen Worten ist das erste Maulteil 5 insgesamt einstückig, was jedoch nicht zwingend erforderlich ist. An dem ersten Maulteil 5 können im Übrigen bspw. eine Elektrode und Isolationskomponenten für das Versiegeln von Körpergewebe angeordnet sein. Der Steuerabschnitt 53 weist bei der gezeigten Ausführungsform neben der Steuernut 51 zudem die komplementäre Schwenklagerfläche 52 und die komplementäre Schwenkführungsfläche 54 auf. Die komplementäre Schwenklagerfläche 52 ist an einer - in Bezug auf die Zeichenebene der Fig. 6 - Oberseite des Steuerabschnitts 53 angeordnet. Die komplementäre Schwenkführungsfläche 54 ist an einer Unterseite 54 angeordnet. Die Steuernut 51 ist zwischen einem proximalen Nutende 511 und einem distalen Nutende 512 längserstreckt. Vorliegend ist die Steuernut 51 zwischen den Nutenden 511, 512 durchgängig gerade und nicht etwa abgewinkelt oder gekrümmt. Letzteres kann bei zeichnerisch nicht dargestellten Ausführungsformen vorgesehen sein. Die Steuernut 51 ist in Bezug auf die Längsachse L2 des ersten Maulteils 5 geneigt längsersteckt. Die Längsneigung verläuft hierbei in distaler Richtung von unten nach oben. Die Längsachse L2 des ersten Maulteils 5 ist jedenfalls in einer Schließstellung des Werkzeugmauls M (Fig. 11) parallel zu der Längsachse L1 des zweiten Maulteils 6 und damit auch parallel zu der Schaftlängsachse L und/oder der Längsbewegungsachse L' orientiert. In der Schließstellung ist die Steuernut 51 um einen Winkel α (Fig. 6) gegenüber den besagten Längsachsen L, L', L1, L2 geneigt. Gegenüber der Längsachse L2 ist der Neigungswinkel α auch in Abhängigkeit der Schwenkstellung des ersten Maulteils 5 um die Schwenkachse S naturgemäß unveränderlich. Dementgegen ändert sich der Neigungswinkel α in Bezug auf die Längsachsen L, L' und L1 in Abhängigkeit der Schwenkstellung.

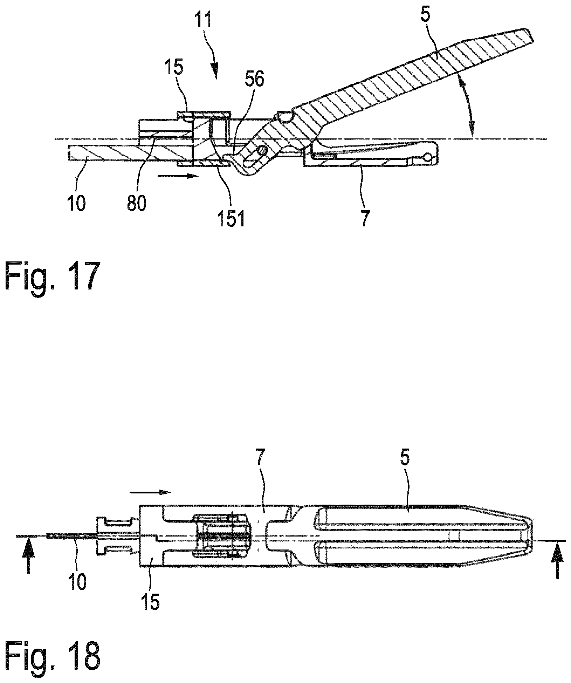

Das erste Maulteil 5 weist vorliegend zudem einen Anschlagabschnitt 56 auf, der zur Begrenzung der Öffnungsstellung mit einem dem zweiten Maulteil 6 zugeordneten komplementären Anschlagabschnitt 151 zusammenwirkt (Fig. 17). Der komplementäre Anschlagabschnitt 151 ist bei der gezeigten Ausführungsform an der Hülse 15 angeordnet. Bei einer zeichnerisch nicht dargestellten Ausführungsform kann der komplementäre Anschlagabschnitt stattdessen unmittelbar an dem Gehäuseabschnitt ausgebildet sein. Der Anschlagabschnitt 56 ragt bei der gezeigten Ausführungsform in proximaler Richtung zapfenförmig von dem Steuerabschnitt 53 ab.

Wie anhand Fig. 7 gezeigt ist, weist das erste Maulteil 5 bei der gezeigten Ausführungsform eine in Bezug auf seine Längsachse L2 spiegelsymmetrische Gestaltung auf. Der Steuerabschnitt 53 weist einen parallel zur Längsachse L2 längserstreckten Aufnahmeschlitz 57 auf, der zur Aufnahme des distalen Endes des Zug-Druck-Elements 10 vorgesehen ist. Der Aufnahmeschlitz 57 untergliedert den Steuerabschnitt 53 und die daran angeordneten Funktionsflächen und/oder Funktionsabschnitte in spiegelsymmetrisch angeordnete und gestaltete Teilflächen und/oder Teilabschnitte. Insoweit kann beispielsweise von der Steuernut 51 und einer weiteren, durch den Aufnahmeschlitz 57 getrennten Steuernut gesprochen werden. Der Einfachheit halber wird nachfolgend jedoch lediglich auf die Steuernut 51 Bezug genommen.

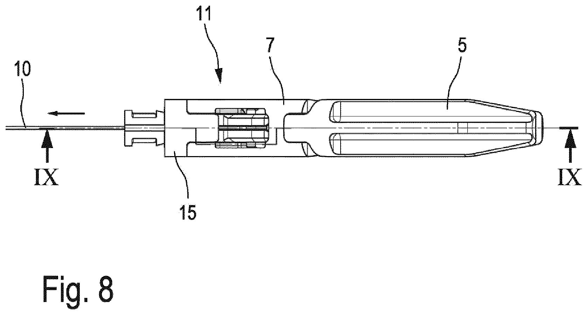

Im betriebsfertig montierten Zustand durchragt der in einer Bohrung 102 (Fig. 2) an dem Zug-Druck-Element 10 radial festgelegte Steuerstift 14 die Steuernut 51 in Axialrichtung A. An seinen axial aus der Steuernut 51 herausragenden Stirnenden wirkt der Steuerstift 14 in radialer Richtung mit den Steuerflächen 74, 74' (Fig. 4) zusammen. Im Rahmen der nachfolgenden Funktionsbeschreibung anhand der Fig. 8 bis 13 wird zur gebotenen Kürze lediglich auf die Steuerfläche 74 bzw. den Steuervorsprung 71 Bezug genommen.

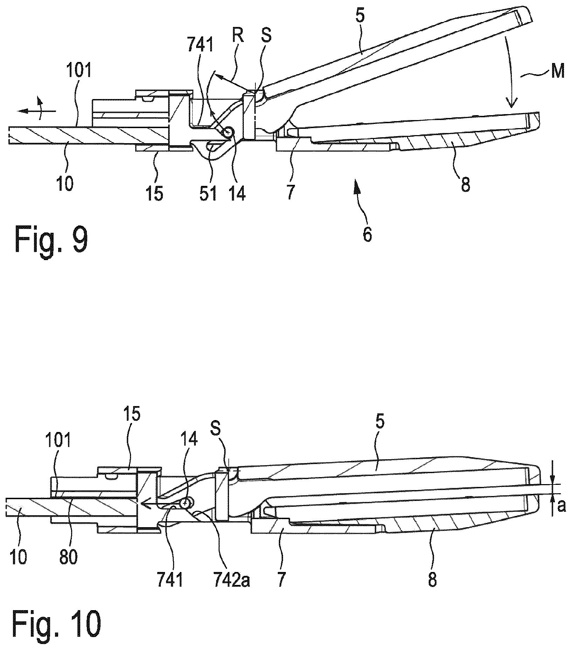

Anhand Fig. 9 ist die Öffnungsstellung des Werkzeugmauls M gezeigt. In dieser ist das erste Maulteil 5 um einen nicht näher bezeichneten Winkel um die Schwenkachse S relativ zu dem zweitem Maulteil 6 verschwenkt, so dass Körpergewebe zwischen den beiden Maulteilen 5, 6, genauer: zwischen den Wirkabschnitten 8, 55, gefasst werden kann. In der Öffnungsstellung des Werkzeugmauls M nimmt der Steuerstift 14 eine distale Stiftendlage ein. Das Bedienelement 9 nimmt die anhand Fig. 1 gezeigte Hubstellung ein. Das Zug-Druck-Element 10 ist entlang der Längsbewegungsachse L' in eine distale Endlage verlagert.

Zur Verlagerung des Werkzeugmauls M in Richtung der Schließstellung (Fig. 11) wird das Bedienelement 9 manuell schwenk- bzw. hubbeweglich in proximaler Richtung verlagert. Die Bedienmechanik 41 und die Koppelmechanik 12 setzen die proximale Hubbewegung des Bedienelements 9 auf grundsätzlich bekannte Weise in eine proximale Längsbewegung des Zug-Druck-Elements 10 um. Hierdurch wird der Steuerstift 14 ausgehend von seiner distalen Stiftendlage (Fig. 9) relativ zu der Steuernut 51 und der Steuerfläche 74 verlagert. In der distalen Stiftendlage ist der Steuerstift 14 vorliegend im Bereich eines distalen Endes des distalen Flächenabschnitts 742a und gleichzeitig im Bereich des distalen Nutendes 512 angeordnet. Von dort bewegt sich der Steuerstift 14 unter Ausübung eines um die Schwenkachse S gerichteten Drehmoments auf das erste Maulteil 5 in proximaler Richtung entlang des distalen Flächenabschnitts 742a. Aufgrund der vorliegenden Längsneigung des distalen Flächenabschnitts 742a wird der Steuerstift 14 hierbei proximal und gleichzeitig - in Bezug auf die Zeichenebene der Fig. 9 bis 13 - in vertikaler Richtung nach oben verlagert. Das Zug-Druck-Element 10 führt hierbei zusätzlich zu seiner translatorischen Bewegung eine geringfügige Rotation um eine nicht näher bezeichnete und parallel zur Schwenkachse S orientierte Schwenkachse im Bereich der Koppelmechanik 42 aus. Aufgrund der mit dem Radius R um die Schwenkachse S konzentrisch gekrümmten Längserstreckung des distalen Flächenabschnitts 742a wird der Steuerstift 14 hierbei auf einer Kreisbahn mit dem Radius R um die Schwenkachse S geführt.

Im Fall der gerade abgewinkelten Führungsbahn nach den Fig. 3 und 4 folgt der Steuerstift 14 nicht der besagten Kreisbahn, sondern wird stattdessen linear entlang des distalen Flächenabschnitts 742 geführt.

In der anhand Fig. 10 gezeigten Funktionsstellung ist der Steuerstift 14 über den distalen Flächenabschnitt 742a hinaus bis in den proximalen Flächenabschnitt 741 verlagert. Das Werkzeugmaul M ist hierbei noch nicht vollständig geschlossen, sondern stattdessen um eine Öffnungsweite a geöffnet. In dieser Funktionsstellung wird vorzugsweise noch keine oder jedenfalls keine wesentliche Klemmkraft auf zwischen den Maulteilen 5, 6 befindliches Körpergewebe ausgeübt. Vielmehr erfolgt der Klemmkraftaufbau ausgehend von der anhand Fig. 10 gezeigten Funktionsstellung. Zum vollständigen Schließen des Werkzeugmauls M und einem damit einhergehenden Klemmkraftaufbau wird der Steuerstift 14 ausgehend von der anhand Fig. 10 gezeigten Stellung weiter in proximaler Richtung verlagert. Hierbei gleitet der Steuerstift 14 entlang des parallel zu der Längsbewegungsachse L' orientierten proximalen Flächenabschnitts 741. Das Zug-Druck-Element 10 wird hierbei gleichzeitig durch die Längsführungsfläche 80 an einer weitergehenden vertikalen Bewegung gehindert.

Anhand Fig. 11 ist die Schließstellung des Werkzeugmauls M gezeigt. In dieser kann ein zusätzlicher Klemmkraftaufbau durch eine weitergehende proximale Verlagerung des Steuerstifts 14 erreicht werden. Durch die parallele Orientierung des proximalen Flächenabschnitts 741 und dessen Positionierung in Relation zu der Schwenkachse S ergeben sich besonders vorteilhafte Hebelverhältnisse zur Kraft- und/oder Drehmomentübertragung auf das erste Maulteil 5. Die vorherrschenden Hebelverhältnisse lassen sich in der anhand Fig. 11 gezeigten Funktionsstellung durch die Hebelarme b, c beschreiben.

Um das Werkzeugmaul M ausgehend von der Schließstellung (Fig. 11) zu öffnen, wird das Bedienelement 9, gegebenenfalls nach vorherigem Lösen der Umlaufsperre 43, distal hub- bzw. schwenkbeweglich verlagert. Das Zug-Druck-Element 10 bewegt sich dementsprechend distal entlang der Längsbewegungsachse L'. Der Steuerstift wird distal entlang des proximalen Flächenabschnitts 741 verlagert (Fig. 12).

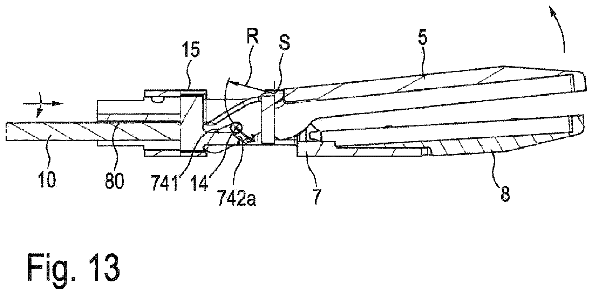

Anhand Fig. 13 ist eine weitere Funktionsstellung beim Öffnen des Werkzeugmauls M gezeigt. In dieser ist der Steuerstift 14 weitergehend in distaler Richtung verlagert und am Übergang zwischen dem proximalen Flächenabschnitt 741 und dem distalen Flächenabschnitt 742a positioniert. Gleichzeitig ist der Steuerstift 14 in dieser Funktionsstellung am distalen Nutende 512 der Steuernut 51 gehalten. Hierdurch wird bei einer weitergehenden Verlagerung des Steuerstifts 14 ein übermäßiges Radialspiel gegenüber dem distalen Flächenabschnitt 742a unterbunden.

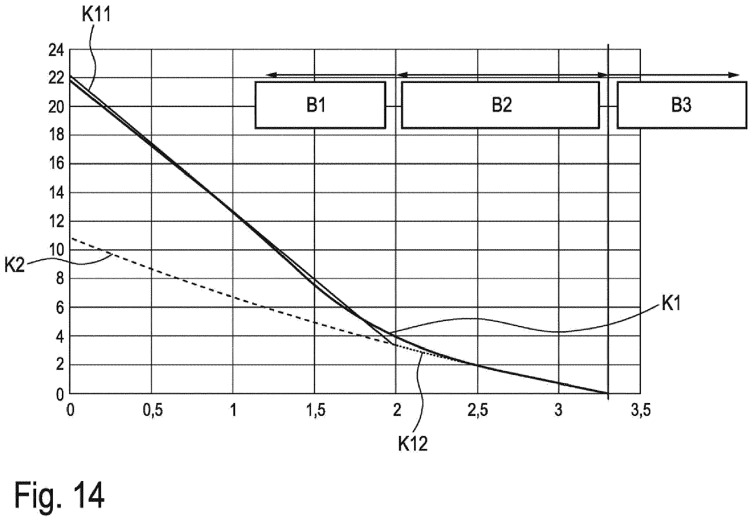

Fig. 14 zeigt ein Diagramm zur Verdeutlichung der Abhängigkeit zwischen einem Öffnungswinkel des Werkzeugmauls M und der translatorischen Verlagerung des Zug-Druck-Elements 10. Letztere kann auch als Hubweg bezeichnet werden und ist auf der Abszisse dargestellt. Der nicht näher bezeichnete Öffnungswinkel des Werkzeugmauls M ist auf der Ordinate dargestellt. Die Kurve K1 gibt den Verlauf des Öffnungswinkels über dem Hubweg für die insbesondere anhand der Fig. 9 bis 13 erläuterte Gestaltung an. Die Kurve K1 lässt sich näherungsweise in zwei lineare Kurvenabschnitte K11, K12 untergliedern. Vorliegend korrespondiert der Koordinatenursprung mit der distalen Stiftendlage und damit der Öffnungsstellung des Werkzeugmauls M (Fig. 9). Der Kurvenverlauf lässt sich in zwei Bereiche B1, B2 untergliedern.

Ausgehend von der distalen Stiftendlage weist der Öffnungswinkel über dem Hubweg zunächst eine vergleichsweise steile Steigung auf (Kurvenabschnitt K11). Hierbei bewegt sich der Steuerstift 14 entlang des distalen Flächenabschnitts 742a. Der Übergang zwischen distalem Flächenabschnitt 742a und proximalem Flächenabschnitt 741 wird vorliegend bei etwa 2 mm Hubweg erreicht. Bei einer weiteren Zunahme des Hubwegs tritt der Steuerstift 14 in den proximalen Flächenabschnitt 741 ein. Der Verlauf des Öffnungswinkels flacht dann merklich ab (Kurvenabschnitt K12).

Dies bedeutet, dass zunächst mit vergleichsweise wenig Hubweg eine vergleichsweise starke Abnahme des Öffnungswinkels erreicht wird (Bereich B1). Da es sich in diesem Bereich um einen anwendungsbedingt niedrigen Kraftbereich handelt (kein Klemmkraftaufbau), hat die Hubverkürzung keine negativen Auswirkungen auf die am Bedienelement 9 erforderlichen Bedienkräfte. Beim Übergang in den Bereich B2 nimmt die Änderung des Öffnungswinkels bei gleicher Änderung des Hubwegs ab. Der eigentliche Aufbau der Klemmkraft zwischen den Maulteilen 5, 6 findet in einem Bereich B3 statt.

Im Ergebnis führt die vorliegende Gestaltung der Steuerfläche 74 zu einem insgesamt verkürzten Hubweg des Bedienelements 9, bei einem größeren Öffnungswinkel des Werkzeugmerkmals M und verringerten Bedienkräften am Bedienelement 9. Hierdurch kann eine verbesserte Anwenderfreundlichkeit erreicht werden.

Hierzu im Unterschied zeigt eine exemplarisch eingezeichnete Kurve K2 den Verlauf des Öffnungswinkels über dem Hubweg für eine rein horizontale Steuerfläche. Bei Verwendung einer rein horizontalen Steuerfläche wird bei gleichem Hubweg nur etwa die Hälfte des Öffnungswinkels erreicht. Dies kann als nachteilig angesehen werden.

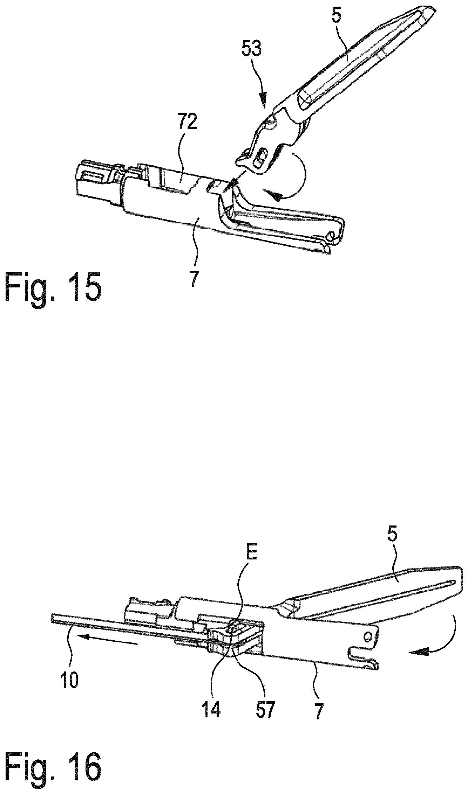

Anhand der Fig. 15 bis 18 sind Verfahrensschritte eines erfindungsgemäßen Verfahrens zur Herstellung der vorbeschriebenen Werkzeugeinrichtung 3 schematisch verdeutlicht.

In einem anhand Fig. 15 verdeutlichten Schritt wird das erste Maulteil 5 mit dem Steuerabschnitt 53 voran in die Aufnahmeaussparung 72 des Gehäuseabschnitts 7 eingeführt. Der Steuerabschnitt 53 wird hierbei schräg von oben nach unten und in proximaler Richtung unter dem Querstegabschnitt 78 hindurchgeführt und gleichzeitig im Uhrzeigersinn gedreht (Fig. 15). Hierbei werden die Schwenklagerfläche 77 und die komplementäre Schwenklagerfläche 52 unter Ausbildung der Schwenkachse S gegeneinander kontaktiert. Zudem werden die Schwenkführungsfläche 79 und die komplementäre Schwenkführungsfläche 54 miteinander kontaktiert. Durch die auf diese Weise ausgebildete einerseits um die Schwenkachse S schwenkbewegliche und andererseits radial zur Schwenkachse S formschlüssige Verbindung zwischen dem ersten Maulteil 5 und dem Gehäuseabschnitt 7 kann insbesondere auf eine gesonderte Stift-, Achs- oder Bolzenverbindung zur Ausbildung der Schwenkachse verzichtet werden.

In einem weiteren Schritt wird das Zug-Druck-Element 10 in den Längsschlitz 57 des Steuerabschnitts 53 eingeführt und der Steuerstift 14 wird entlang seiner Axialrichtung durch die Steuernut 51 und die Aufnahmebohrung 102 gesteckt. Dies erfolgt bei einer maximalen Öffnung des ersten Maulteils gegenüber dem Gehäuseabschnitt 7, die auch als Montagestellung bezeichnet werden kann (Fig. 16). Der Steuerstift 14 befindet sich hierbei noch außerhalb der Aufnahmeaussparung 72 und im Bereich einer distal endseitig an dem Steuervorsprung 71 ausgebildeten Einführöffnung E.

Ausgehend von der Montagestellung wird das erste Maulteil 5 manuell im Uhrzeigersinn um die Schwenkachse verschwenkt, so dass der Steuerstift 14 - bei einer entsprechenden Positionierung - durch die Einführöffnung E in die Aufnahmeaussparung 72 und in den Bereich der Steuerfläche 74 gelangt. Hierbei wird der Steuerstift 14 entlang seiner axialen Richtung zwischen den gegenüberliegenden Sicherungsflächen 76, 76' geführt.

In einem weiteren Schritt (Fig. 17, 18) wird die Hülse 15 in axialer Richtung über das Zug-Druck-Element 10 und das proximale Ende des Gehäuseabschnitts 7 geschoben. Hierdurch wird das Zug-Druck-Element 10 in seiner vertikalen Beweglichkeit zwischen einer nicht näher bezeichneten Innenseite der Hülse 15 und der Längsführungsfläche 80 des Gehäuseabschnitts 7 festgelegt. Dabei kann sich das Zug-Druck-Element 10 bestimmungsgemäß begrenzt in vertikaler Richtung zwischen der Innenseite der Hülse 15 und der Längsführungsfläche 80 bewegen. Durch den Anschlagabschnitt 56 und den komplementären Anschlagabschnitt 151 der Hülse 15 wird die Schwenkbeweglichkeit des ersten Maulteils 5 einseitig formschlüssig begrenzt. Hierdurch wird verhindert, dass das erste Maulteil 5 über die Öffnungsstellung (Fig. 17) hinweg in die Montagestellung (Fig. 16) gelangen kann und der Steuerstift 14 ungewollt aus der Aufnahmeaussparung 72 austritt. Mit anderen Worten wird die Steueranordnung 11 durch die Hülse 15 in sich fixiert.

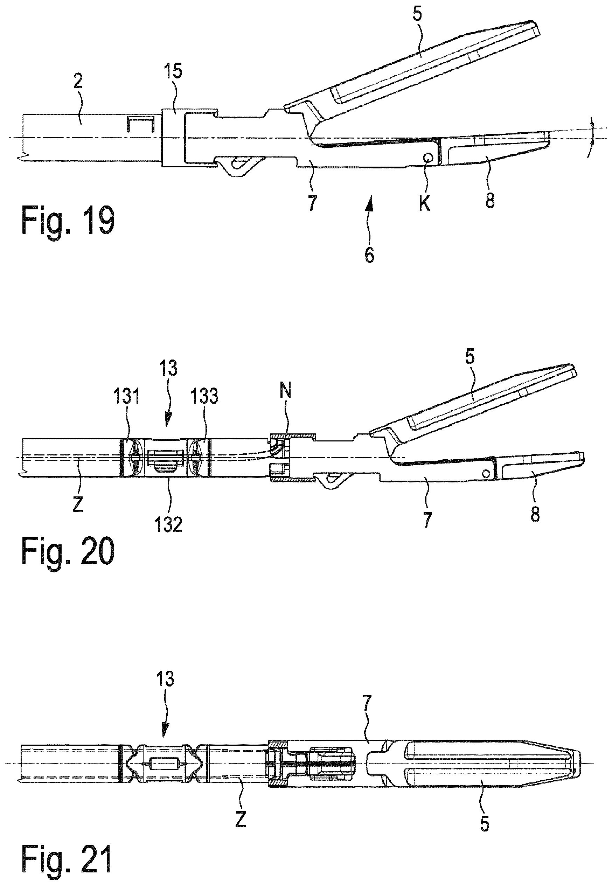

In einem nächsten Schritt (Fig. 19) wird der Schaft 2 in axialer Richtung über das Zug-Druck-Element 10 geführt und an dem Gehäuseabschnitt 7 festgelegt. Hierbei wird eine Crimpverbindung zwischen dem Gehäuseabschnitt 7 und dem Schaft 2 ausgebildet. Der Gehäuseabschnitt 7 weist hierfür nicht näher bezeichnete Vertiefungen auf (bspw. Fig 4), die mit an dem distalen Ende des Schafts 2 angeordneten Laschen (ohne Bezugszeichen) zusammenwirken. Infolge der Crimpverbindung wird die Hülse 15 - vereinfacht ausgedrückt - zwischen dem Schaft 2 und dem Gehäuseabschnitt 7 "eingeklemmt".

In einem weiteren Schritt wird der Wirkabschnitt 8 des zweiten Maulteils 6 mit einer weiteren formschlüssigen Verbindung im Bereich der Kippachse K an dem Gehäuseabschnitt 8 festgelegt. Wie Fig. 19 verdeutlicht, ist der Wirkabschnitt 8 bei der vorliegenden Ausführungsform relativ zu dem Gehäuseabschnitt 7 um wenige Winkelgrade um die Kippachse K kippbeweglich.

Die anhand Fig. 19 verdeutlichten Schritte beziehen sich auf die Variante mit gegenüber dem Schaft 2 nicht abwinkelbarer Werkzeugeinrichtung. Die abwinkelbare Variante ist anhand der Fig. 20 und 21 gezeigt. Bei dieser Variante ist ein Artikulationszugband Z vorgesehen. Das Artikulationszugband Z ist in überdeckten Bereichen gestrichelt dargestellt und wirkt auf grundsätzlich bekannte Weise mit der Bedieneinheit 12 und der Wirbeleinheit 13 des Abwinkelungsmechanismus 12, 13 zusammen. Das Artikulationszugband Z verläuft entlang einer nicht näher bezeichneten Nut in dem Gehäuseabschnitt 7 und als Schleife entlang des Schafts 2. Einzelne Wirbelkörper 131, 132, 133 der Wirbeleinrichtung 13 gestatten eine Abwinkelung der Werkzeugeinrichtung 3. Dabei wird die anhand der Fig. 20 und 21 ersichtliche Anordnung vorzugsweise ausschließlich über die Zugkräfte des Artikulationszugbands Z zusammengehalten, so dass keine weitere Formverbindung zur Fixierung der Anordnung benötigt wird. Die Hülse 15 sichert das Artikulationszugband Z im Bereich einer Nut N des Gehäuseabschnitts 7 in allen Richtungen gegen ein Herausrutschen.

## Patentansprüche

1. Werkzeugeinrichtung (3) für ein chirurgisches Instrument (1), mit einem ersten Maulteil (5) und einem zweiten Maulteil (6), wobei das erste und zweite Maulteil (5, 6) unter Ausbildung eines öffenbaren und schließbaren Werkzeugmauls (M) um eine Schwenkachse (S) relativ zueinander schwenkbeweglich sind, wobei die Werkzeugeinrichtung (3) eine Steueranordnung (11) mit einem Steuerstift (14) und wenigstens einer Steuernut (51) aufweist, wobei die wenigstens eine Steuernut (51) an einem Steuerabschnitt (53) des ersten Maulteils (5) ausgebildet ist, wobei der Steuerstift (14) parallel zu der Schwenkachse (S) längserstreckt ist und die wenigstens eine Steuernut (51) axial durchgreift, wobei der Steuerstift (14) zwischen einer proximalen Stiftendlage und einer distalen Stiftendlage entlang der Steuernut (51) gleitbeweglich verlagerbar ist und hierbei zum Öffnen und/oder Schließen des Werkzeugmauls (M) ein um die Schwenkachse (S) wirkendes Drehmoment auf das erste Maulteil (5) bewirkt, und wobei das zweite Maulteil (6) eine Aufnahmeaussparung (72) aufweist, in welcher der Steuerabschnitt (53) des ersten Maulteils (5) wenigstens abschnittsweise aufgenommen ist, **dadurch gekennzeichnet, dass** wenigstens ein Steuervorsprung (71) von einer Innenwandung (73) der Aufnahmeaussparung (72) in Axialrichtung (A) des Steuerstifts (14) nach innen abragt und eine Steuerfläche (74) der Steueranordnung (11) ausbildet, und wobei der Steuerstift (14) bei einer Verlagerung zwischen der proximalen und distalen Stiftendlage entlang des Steuervorsprungs (71) gleitet und radial auf demselben abgestützt ist.

2. Werkzeugeinrichtung (3) nach Anspruch 1, **dadurch gekennzeichnet, dass** das zweite Maulteil (6) die Aufnahmeaussparung (72) in Axialrichtung (A) des Steuerstifts (14) begrenzende Sicherungsfläche (76, 76') aufweist, zwischen welchen der Steuerstift (14) bei einer Verlagerung zwischen der proximalen und distalen Stiftendlage gegen eine axiale Verschiebung formschlüssig gesichert ist.

3. Werkzeugeinrichtung (3) nach Anspruch 1 oder 2, **dadurch gekennzeichnet, dass** das zweite Maulteil (6) eine Schwenklagerfläche (77) aufweist, die unter Ausbildung der Schwenkachse (S) mit einer komplementären Schwenklagerfläche (52) des ersten Maulteils (5) gleitbeweglich zusammenwirkt.

4. Werkzeugeinrichtung (3) nach Anspruch 3, **dadurch gekennzeichnet, dass** die Schwenklagerfläche (77) des zweiten Maulteils (6) durch einen Außenumfang eines Querstegabschnitts (78) gebildet ist, welcher die Aufnahmeaussparung (72) in Axialrichtung (A) des Steuerstifts (14) übergreift.

5. Werkzeugeinrichtung (3) nach einem der vorhergehenden Ansprüche, **dadurch gekennzeichnet, dass** das zweite Maulteil (6) eine Schwenkführungsfläche (79) aufweist, welche gekrümmt und konzentrisch zu der Schwenkachse (S) längserstreckt ist, und welche mit einer komplementären Schwenkführungsfläche (54) des ersten Maulteils gleitbeweglich zusammenwirkt.

6. Werkzeugeinrichtung (3) nach einem der vorhergehenden Ansprüche, **dadurch gekennzeichnet, dass** ein an dem zweiten Maulteil (6) angeordneter und/oder ausgebildeter Anschlagabschnitt (151) vorgesehen ist, welcher in einer Öffnungsstellung des Werkzeugmauls (M) mit einem komplementären Anschlagabschnitt (56) des ersten Maulteils (5) um die Schwenkachse (S) formschlüssig zusammenwirkt.

7. Werkzeugeinrichtung (3) nach einem der vorhergehenden Ansprüche, **dadurch gekennzeichnet, dass** das zweite Maulteil (6) in Bezug auf die Schwenkachse (S) feststehend ist, und dass das erste Maulteil (5) beim Öffnen und Schließen - des Werkzeugmauls (M) relativ zu dem zweiten Maulteil (6) um die Schwenkachse (S) rotiert.

8. Werkzeugeinrichtung (3) nach einem der vorhergehenden Ansprüche, **dadurch gekennzeichnet, dass** das zweite Maulteil (6) einen einstückigen Gehäuseabschnitt (7) und einen distal an dem Gehäuseabschnitt (7) montierten Wirkabschnitt (8) aufweist, wobei der Gehäuseabschnitt (7) wenigstens die Aufnahmeaussparung (72) mitsamt des wenigstens einen Steuervorsprungs (71) und/oder die Schwenklagerfläche (77) aufweist.

9. Werkzeugeinrichtung (3) nach einem der vorhergehenden Ansprüche, **dadurch gekennzeichnet, dass** die Steuernut (51) zwischen einem proximalen Nutende (511) und einem distalen Nutende (512) durchgängig gerade längserstreckt ist.

10. Chirurgisches Instrument (1) aufweisend
einen entlang einer Schaftlängsachse (L) längserstreckten Schaft (2),
eine distal an dem Schaft (2) angeordnete Werkzeugeinrichtung (3) nach einem der Ansprüche 1 bis 9,
eine proximal an dem Schaft (2) angeordnete Griffeinrichtung (4) mit einem Bedienelement (9), welches über ein Zug-Druck-Element (10) mit der Werkzeugeinrichtung (3) wirkverbunden ist, wobei das Zug-Druck-Element (10) mittels einer Bedienung des Bedienelements (9) entlang einer Längsbewegungsachse (L') translatorisch verlagerbar ist,
wobei der Steuerstift (14) der Werkzeugeinrichtung (3) mittels einer Längsbewegung des Zug-Druck-Elements (10) zwischen einer proximalen Stiftendlage und einer distalen Stiftendlage entlang der Steuernut (51) gleitbeweglich verlagerbar ist und hierbei zum Öffnen und/oder Schließen des Werkzeugmauls (M) ein um die Schwenkachse (S) wirkendes Drehmoment auf das erste Maulteil (5) bewirkt.

11. Chirurgisches Instrument (1) nach Anspruch 10, **dadurch gekennzeichnet, dass** das zweite Maulteil (6) eine Längsführungsfläche (80) aufweist, welche parallel zu der Längsbewegungsachse (L') des Zug-Druck-Elements (10) längserstreckt ist, und an welcher eine komplementäre Längsführungsfläche (101) des Zug-Druck-Elements (10) bei dessen Längsbewegung wenigstens zeitweise gleitbeweglich geführt ist.

12. Chirurgisches Instrument (1) nach Anspruch 10 oder 11, **dadurch gekennzeichnet, dass** die Steuerfläche (74) einen proximalen Flächenabschnitt (741) und einen distalen Flächenabschnitt (742, 742a) aufweist, welche relativ zu der Längsbewegungsachse (L') derart unterschiedlich geneigt längserstreckt sind, dass bei einer Verlagerung des Steuerstifts (14) zwischen der proximalen und der distalen Stiftendlage unterschiedliche Übersetzungsverhältnisse zwischen der Längsbewegung des Zug-Druck-Elements (10) und der Schwenkbewegung des Werkzeugmauls (M) erreicht sind.

13. Chirurgisches Instrument (1) nach Anspruch 12, **dadurch gekennzeichnet, dass** der proximale Flächenabschnitt (741) parallel und/oder wenigstens im Wesentlichen parallel zu der Längsbewegungsachse (L') längserstreckt ist.

14. Chirurgisches Instrument (1) nach Anspruch 12 oder 13, **dadurch gekennzeichnet, dass** der distale Flächenabschnitt (742a) gekrümmt und konzentrisch zu der Schwenkachse (S) längserstreckt ist.

15. Chirurgisches Instrument (1) nach Anspruch 12 oder 13, **dadurch gekennzeichnet, dass** der distale Flächenabschnitt (742) gerade und tangential zu einem konzentrisch zu der Schwenkachse (S) angeordneten Kreisbogen längserstreckt ist.

16. Chirurgisches Instrument (1) nach einem der Ansprüche 11 bis 15, **dadurch gekennzeichnet, dass** der Steuerstift (14) bei einer Verlagerung in Richtung der distalen Stiftendlage wenigstens zeitweise an dem distalen Nutende (512) der Steuernut (51) gehalten ist und gemeinsam mit dem ersten Maulteil (5) um die Schwenkachse (S) rotiert.

17. Verfahren zur Herstellung einer Werkzeugeinrichtung (3) nach Anspruch 1 aufweisend den Schritt: Urformen des zweiten Maulteils (6), wobei wenigstens ein den wenigstens einen Steuervorsprung (71) tragender Gehäuseabschnitt (7) des zweiten Maulteils (6) urgeformt wird.

18. Verfahren nach Anspruch 17, aufweisend den Schritt: formschlüssiges Zusammenfügen des ersten Maulteils (5) und des urgeformten Gehäuseabschnitts (7), wobei eine an dem Gehäuseabschnitt (7) ausgebildete Schwenklagerfläche (77) des zweiten Maulteils (6) - und eine komplementäre Schwenklagerfläche (52) des ersten Maulteils (5) unter Ausbildung der Schwenkachse (S) um dieselbe gleitbeweglich und radial zu der Schwenkachse (S) formschlüssig aneinander festgelegt werden.

## Claims

1. Tool device (3) for a surgical instrument (1), having a first jaw part (5) and a second jaw part (6), the first and second jaw parts (5, 6) being pivotable relative to each other, about a pivot axis (S), to form an openable and closable tool mouth (M), the tool device (3) having a control arrangement (11) with a control pin (14) and at least one control groove (51), the at least one control groove (51) being formed on a control portion (53) of the first jaw part (5), the control pin (14) extending parallel to the pivot axis (S) and engaging axially through the at least one control groove (51), the control pin (14) being slidably movable between a proximal end position of the pin and a distal end position of the pin along the control groove (51) and in this way applying to the first jaw part (5) a torque acting about the pivot axis (S) in order to open and/or close the tool mouth (M), and the second jaw part (6) having a receiving recess (72) in which the control portion (53) of the first jaw part (5) is received at least in part, **characterized in that** at least one control projection (71) protrudes inward from an inner wall (73) of the receiving recess (72) in the axial direction (A) of the control pin (14) and forms a control surface (74) of the control arrangement (11), and wherein the control pin (14), in a movement between the proximal and distal end positions of the pin, slides along the control projection (71) and is supported radially on the latter.

2. Tool device (3) according to Claim 1, **characterized in that** the second jaw part (6) has securing surfaces (76, 76') which delimit the receiving recess (72) in the axial direction (A) of the control pin (14) and between which the control pin (14), in a movement between the proximal and distal end positions of the pin, is positively secured against axial displacement.

3. Tool device (3) according to Claim 1 or 2, **characterized in that** the second jaw part (6) has a pivot bearing surface (77) which, forming the pivot axis (S), interacts in a sliding movement with a complementary pivot bearing surface (52) of the first jaw part (5).

4. Tool device (3) according to Claim 3, **characterized in that** the pivot bearing surface (77) of the second jaw part (6) is formed by an outer circumference of a transverse web portion (78) which engages over the receiving recess (72) in the axial direction (A) of the control pin (14).

5. Tool device (3) according to one of the preceding claims, **characterized in that** the second jaw part (6) has a pivot guiding surface (79) which is curved and concentric with respect to the pivot axis (S) and which interacts in a sliding movement with a complementary pivot guiding surface (54) of the first jaw part.

6. Tool device (3) according to one of the preceding claims, **characterized in that** a stop portion (151) arranged and/or formed on the second jaw part (6) is provided which, in an open position of the tool mouth (M), interacts positively with a complementary stop portion (56) of the first jaw part (5) about the pivot axis (S).

7. Tool device (3) according to one of the preceding claims, **characterized in that** the second jaw part (6) is fixed in relation to the pivot axis (S), and **in that** the first jaw part (5), during the opening and closing of the tool mouth (M), rotates about the pivot axis (S) relative to the second jaw part (6).

8. Tool device (3) according to one of the preceding claims, **characterized in that** the second jaw part (6) has a one-piece housing portion (7) and an active portion (8) mounted distally on the housing portion (7), the housing portion (7) having at least the receiving recess (72) together with the at least one control projection (71) and/or the pivot bearing surface (77).

9. Tool device (3) according to one of the preceding claims, **characterized in that** the control groove (51) between a proximal groove end (511) and a distal groove end (512) extends continuously straight.

10. Surgical instrument (1) having
a shaft (2) extending along a shaft longitudinal axis (L),
a tool device (3) according to one of Claims 1 to 9 arranged distally on the shaft (2),
a grip device (4) arranged proximally on the shaft (2) and having an operating element (9) which is operatively connected to the tool device (3) via a push-pull element (10), the push-pull element (10) being movable in translation along a longitudinal movement axis (L') by means of an operation of the operating element (9),
the control pin (14) of the tool device (3) being slidably movable, by means of a longitudinal movement of the pull-push element (10), between a proximal end position of the pin and a distal end position of the pin along the control groove (51) and in this way applying to the first jaw part (5) a torque acting about the pivot axis (S) in order to open and/or close the tool mouth (M).

11. Surgical instrument (1) according to Claim 10, **characterized in that** the second jaw part (6) has a longitudinal guide surface (80) which extends parallel to the longitudinal movement axis (L') of the push-pull element (10) and on which a complementary longitudinal guide surface (101) of the push-pull element (10) is at least temporarily guided slidably during the longitudinal movement of the latter.

12. Surgical instrument (1) according to Claim 10 or 11, **characterized in that** the control surface (74) has a proximal surface portion (741) and a distal surface portion (742, 742a) which are inclined differently relative to the longitudinal movement axis (L') in such a way that, in a movement of the control pin (14) between the proximal and the distal end positions of the pin, different transmission ratios between the longitudinal movement of the push-pull element (10) and the pivoting movement of the tool mouth (M) are achieved.

13. Surgical instrument (1) according to Claim 12, **characterized in that** the proximal surface portion (741) extends parallel and/or at least substantially parallel to the longitudinal movement axis (L').

14. Surgical instrument (1) according to Claim 12 or 13, **characterized in that** the distal surface portion (742a) is curved and concentric with respect to the pivot axis (S) .

15. Surgical instrument (1) according to Claim 12 or 13, **characterized in that** the distal surface portion (742) is straight and tangential to a circular arc arranged concentrically with respect to the pivot axis (S).

16. Surgical instrument (1) according to one of Claims 11 to 15, **characterized in that** the control pin (14), in a movement in the direction of the distal end position of the pin, is held at least temporarily at the distal groove end (512) of the control groove (51) and rotates together with the first jaw part (5) about the pivot axis (S) .

17. Method for producing a tool device (3) according to Claim 1, comprising the step of primary forming of the second jaw part (6), wherein at least one housing portion (7) of the second jaw part (6) carrying the at least one control projection (71) is provided by primary forming.

18. Method according to Claim 17, comprising the step of positively joining together the first jaw part (5) and the primary-formed housing portion (7), wherein a pivot bearing surface (77) of the second jaw part (6) formed on the housing portion (7) and a complementary pivot bearing surface (52) of the first jaw part (5), forming the pivot axis (S), are slidably movable about the latter and are secured form-fittingly to each other radially with respect to the pivot axis (S).

## Revendications

1. Dispositif d'outil (3) pour un instrument chirurgical (1), avec une première partie de mâchoire (5) et une deuxième partie de mâchoire (6), la première et la deuxième parties de mâchoire (5, 6) étant mobiles en pivotement l'une par rapport à l'autre autour d'un axe de pivotement (S) en formant une mâchoire d'outil (M) étant apte à être ouverte et fermée, le dispositif d'outil (3) présentant un agencement de commande (11) avec une broche de commande (14) et au moins une rainure de commande (51), l'au moins une rainure de commande (51) étant formée sur une section de commande (53) de la première partie de mâchoire (5), la broche de commande (14) s'étendant longitudinalement parallèlement à l'axe de pivotement (S) et traversant axialement l'au moins une rainure de commande (51), la broche de commande (14) étant apte à être déplacée de manière coulissante le long de la rainure de commande (51) entre une position extrême proximale de la broche et une position extrême distale de la broche et provoquant ainsi, pour l'ouverture et/ou la fermeture de la mâchoire d'outil (M), un couple agissant autour de l'axe de pivotement (S) sur la première partie de mâchoire (5), et la deuxième partie de mâchoire (6) présentant un évidement de réception (72) dans lequel la section de commande (53) de la première partie de mâchoire (5) est reçue au moins partiellement, **caractérisé en ce qu'**au moins une saillie de commande (71) fait saillie vers l'intérieur d'une paroi longitudinale (73) de l'évidement de réception (72) dans la direction axiale (A) de la broche de commande (14) et forme une surface de commande (74) de l'agencement de commande (11), et la broche de commande (14) coulissant le long de la saillie de commande (71) lors d'un déplacement entre les positions extrêmes proximale et distale de la broche et s'appuyant radialement sur celle-ci.

2. Dispositif d'outil (3) selon la revendication 1, **caractérisé en ce que** la deuxième partie de mâchoire (6) présente des surfaces de blocage (76, 76') délimitant l'évidement de réception (72) dans la direction axiale (A) de la broche de commande (14), entre lesquelles la broche de commande (14) est bloquée par complémentarité de forme à l'égard d'un déplacement axial lors d'un déplacement entre les positions extrêmes proximale et distale de la broche.

3. Dispositif d'outil (3) selon la revendication 1 ou 2, **caractérisé en ce que** la deuxième partie de mâchoire (6) présente une surface (77) formant palier de pivotement qui, en formant l'axe de pivotement (S), coopère de manière coulissante avec une surface (52) formant palier de pivotement complémentaire de la première partie de mâchoire (5).

4. Dispositif d'outil (3) selon la revendication 3, **caractérisé en ce que** la surface (77) formant palier de pivotement de la deuxième partie de mâchoire (6) est formée par une périphérie extérieure d'une partie de barrette transversale (78) qui est en chevauchement de l'évidement de réception (72) dans la direction axiale (A) de la broche de commande (14).

5. Dispositif d'outil (3) selon l'une des revendications précédentes, **caractérisé en ce que** la deuxième mâchoire (6) présente une surface (79) de guidage en pivotement qui est incurvée et allongée concentriquement à l'axe de pivotement (S), et qui coopère par coulissement avec une surface (54) de guidage en pivotement complémentaire de la première mâchoire.

6. Dispositif d'outil (3) selon l'une des revendications précédentes, **caractérisé en ce qu'**il est prévu une partie de butée (151) disposée et/ou formée sur la deuxième partie de mâchoire (6), qui, dans une position d'ouverture de la mâchoire d'outil (M), coopère par complémentarité de forme avec une partie de butée (56) complémentaire de la première partie de mâchoire (5) autour de l'axe de pivotement (S).

7. Dispositif d'outil (3) selon l'une des revendications précédentes, **caractérisé en ce que** la deuxième partie de mâchoire (6) est fixe par rapport à l'axe de pivotement (S), et **en ce que** la première partie de mâchoire (5) tourne autour de l'axe de pivotement (S) par rapport à la deuxième partie de mâchoire (6) lors de l'ouverture et de la fermeture de la mâchoire d'outil (M).

8. Dispositif d'outil (3) selon l'une des revendications précédentes, **caractérisé en ce que** la deuxième partie de mâchoire (6) présente une section de boîtier (7) d'une seule pièce et une section active (8) montée de manière distale sur la section de boîtier (7), la section de boîtier (7) présentant au moins l'évidement de réception (72) y compris l'au moins une saillie de commande (71) et/ou la surface (77) formant palier de pivotement.

9. Dispositif d'outil (3) selon l'une des revendications précédentes, **caractérisé en ce que** la rainure de commande (51) s'étend longitudinalement de façon rectiligne et continue entre une extrémité proximale (511) de rainure et une extrémité distale (512) de rainure.

10. Instrument chirurgical (1) comprenant
une tige (2) s'étendant longitudinalement le long d'un axe longitudinal (L) de la tige,
un dispositif d'outil (3) disposé de manière distale sur la tige (2) selon l'une des revendications 1 à 9,
un dispositif de préhension (4) disposé de manière proximale sur la tige (2), avec un élément de commande (9) qui est en liaison active avec le dispositif d'outil (3) par l'intermédiaire d'un élément de traction-pression (10), l'élément de traction-pression (10) étant apte à être déplacé en translation le long d'un axe de déplacement longitudinal (L') au moyen d'une commande de l'élément de commande (9), la broche de commande (14) du dispositif d'outil (3) étant apte à être déplacée de manière coulissante le long de la rainure de commande (51) au moyen d'un mouvement longitudinal de l'élément de traction-pression (10) entre une position extrême proximale de la broche et une position extrême distale de la broche et provoquant ainsi un couple de rotation agissant autour de l'axe de pivotement (S) sur la première partie de mâchoire (5) pour ouvrir et/ou fermer la mâchoire d'outil (M).

11. Instrument chirurgical (1) selon la revendication 10, **caractérisé en ce que** la deuxième partie de mâchoire (6) présente une surface de guidage longitudinale (80) qui s'étend longitudinalement parallèlement à l'axe de déplacement longitudinal (L') de l'élément de traction-pression (10) et sur laquelle une surface de guidage longitudinale complémentaire (101) de l'élément de traction-pression (10) est guidée de manière coulissante au moins temporairement lors de son déplacement longitudinal.

12. Instrument chirurgical (1) selon la revendication 10 ou 11, **caractérisé en ce que** la surface de commande (74) présente une partie de surface proximale (741) et une partie de surface distale (742, 742a), qui s'étendent longitudinalement avec une inclinaison différente par rapport à l'axe de mouvement longitudinal (L') de telle sorte que, lors d'un déplacement de la broche de commande (14) entre la position extrême proximale et la position extrême distale de la broche, des rapports de transmission différents sont obtenus entre le mouvement longitudinal de l'élément de traction-pression (10) et le mouvement de pivotement de la mâchoire d'outil (M).

13. Instrument chirurgical (1) selon la revendication 12, **caractérisé en ce que** la partie de surface proximale (741) est allongée parallèlement et/ou au moins sensiblement parallèlement à l'axe de déplacement longitudinal (L').

14. Instrument chirurgical (1) selon la revendication 12 ou 13, **caractérisé en ce que** la partie de surface distale (742a) est incurvée et s'étend longitudinalement de manière concentrique à l'axe de pivotement (S).

15. Instrument chirurgical (1) selon la revendication 12 ou 13, **caractérisé en ce que** la partie de surface distale (742) est droite et s'étend longitudinalement de façon tangentielle à un arc de cercle agencé concentriquement à l'axe de pivotement (S).

16. Instrument chirurgical (1) selon l'une des revendications 11 à 15, **caractérisé en ce que** la broche de commande (14) est maintenue au moins temporairement à l'extrémité distale (512) de la rainure de commande (51) lors d'un déplacement en direction de la position extrême distale de la broche et tourne conjointement avec la première partie de mâchoire (5) autour de l'axe de pivotement (S).

17. Procédé de fabrication d'un dispositif d'outil (3) selon la revendication 1, comprenant l'étape consistant à : former la deuxième partie de mâchoire (6), au moins une section de boîtier (7) de la deuxième partie de mâchoire (6), qui porte l'au moins une saillie de commande (71), étant formée.

18. Procédé selon la revendication 17, comprenant l'étape consistant à : assembler par complémentarité de forme la première partie de mâchoire (5) et la partie de boîtier (7) préformée, une surface (77) qui forme palier de pivotement de la deuxième partie de mâchoire (6) formée sur la section de boîtier (7) et une surface (52) qui forme palier de pivotement complémentaire de la première partie de mâchoire (5) étant fixées l'une à l'autre par complémentarité de forme en formant l'axe de pivotement (S) en étant apte à coulisser autour de celui-ci et radialement par rapport à l'axe de pivotement (S).
